(19) Europäisches Patentamt
European Patent Office
Office européen des brevets

(11) Publication number: **0 433 662 A2**

(12) **EUROPEAN PATENT APPLICATION**

(21) Application number: **90121904.8**

(22) Date of filing: **15.11.90**

(51) Int. Cl.[5]: **C07C 233/07**, A61K 31/165, C07D 295/185, C07D 213/40, C07D 211/16, C07D 211/22, C07D 233/18, C07C 233/13, C07C 233/36, C07C 275/16, C07C 237/22

(30) Priority: **16.11.89 US 437727**
**09.10.90 US 594484**

(43) Date of publication of application:
**26.06.91 Bulletin 91/26**

(84) Designated Contracting States:
**AT BE CH DE DK ES FR GB GR IT LI LU NL SE**

(71) Applicant: **WARNER-LAMBERT COMPANY**
**201 Tabor Road**
**Morris Plains New Jersey 07950(US)**

(72) Inventor: **Roark, William Howard**
**2810 Gladstone**
**Ann Arbor, Michigan 48104(US)**

(74) Representative: **Mansmann, Ivo**
**c/o Gödecke AG - Patentabteilung Postfach**
**569 Mooswaldallee 1-9**
**W-7800 Freiburg(DE)**

(54) **Acat inhibitors.**

(57) The invention is novel compounds having acyl-coenzyme A: cholesterol acyltransferase (ACAT) inhibitory activity of the formula I

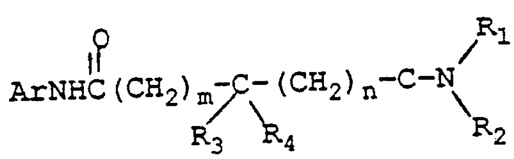

(I),

The invention further comprises a process for the preparation of compounds of the formula I, pharmaceutical compositions containing them and the use of compounds (I) for the preparation of pharmaceutical compositions useful for treating hypercholesterolemia and atherosklerosis.

EP 0 433 662 A2

# ACAT INHIBITORS

## BACKGROUND OF THE INVENTION

This invention relates to chemical compounds having pharmacological activity, to pharmaceutical compositions which include these compounds, and to the use of the compounds for the preparation of pharmaceuticals. More particularly, this invention concerns certain novel compounds which inhibit the enzyme acylcoenzyme A: cholesterol acyltransferase (ACAT), pharmaceutical compositions containing these compounds, and the use of the compounds for the preparation of pharmaceuticals useful for treating hypercholesterolemia and atherosclerosis.

In recent years the role which elevated blood plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which could be effective in lowering total serum cholesterol levels. It is now known that cholesterol is transported in the blood in the form of complex particles consisting of a core of cholesteryl esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipoprotein cholesterol (HDL cholesterol).

Following these discoveries, the search for therapeutic agents which control serum cholesterol turned to finding compounds which are more selective in their action; that is, agents which are effective in elevating the blood serum levels of HDL cholesterol and/or lowering the levels of LDL cholesterol. While such agents are effective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of the enzyme acyl-CoA: cholesterol acyltransferase (ACAT) before it can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

## SUMMARY OF THE INVENTION

The present invention provides a class of compounds which have acyl-CoA: cholesterol acyltransferase (ACAT) inhibitory activity and intermediates useful in preparing said compounds having the following structure:

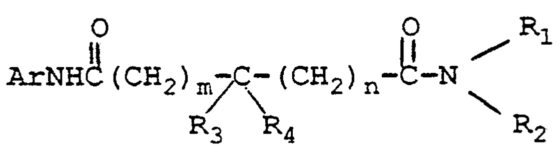

I

wherein Ar is

(a) phenyl$(CH_2)_x$- wherein x is zero to 2 and wherein the phenyl ring is unsubstituted or is substituted with from 1 to 3 substituents selected from

alkyl having from 1 to 6 carbon atoms and which is straight or branched,
alkoxy having from 1 to 6 carbon atoms and which is straight or branched,
phenoxy,
hydroxy,
fluorine,
chlorine,
bromine,

nitro,
trifluoromethyl,
-COOH,
-COOalkyl wherein alkyl has from 1 to 4 carbon atoms,
$-NR_5R_6$ wherein
$R_5$ and $R_6$ are independently hydrogen or alkyl of from 1 to 4 carbon atoms;
(b) 1- or 2-naphthyl which is unsubstituted or substituted with
alkyl having from 1 to 6 carbon atoms and which is straight or branched;
alkoxy having from 1 to 6 carbon atoms and which is straight or branched,
hydroxy,
fluorine,
chlorine,
bromine,
nitro,
trifluoromethyl,
-COOH,
-COOalkyl wherein alkyl has from 1 to 4 carbon atoms,
$-NR_5R_6$ wherein $R_5$ and $R_6$ have the meanings defined above;
wherein each of m and n is independently zero, one, or two;
wherein each of $R_3$ and $R_4$ is independently
(a) hydrogen;
(b) a straight or branched alkyl group having from 1 to 10 carbon atoms;
(c) a straight chain alkyl group having from 1 to 10 carbon atoms wherein the terminal carbon atom is substituted with hydroxy or $NR_5R_6$ wherein $R_5$ and $R_6$ have the meanings defined above; or
(d) $R_3$ is hydrogen and $R_4$ is the group $-NHR_{10}$ wherein $R_{10}$ is -C(=O)alkyl $C_{1-6}$ wherein the alkyl moiety is straight or branched; $-(C=O)O\text{-t-}C_4H_9$; $-C(=O)OCH_2C_6H_5$;

$$-\overset{\overset{X}{\|}}{C}NH\text{alkyl } C_{1-6}$$

wherein X is oxygen or sulfur and the alkyl moiety is straight or branched;

$$-\overset{\overset{X}{\|}}{C}NHPh$$

wherein X is oxygen or sulfur and Ph is phenyl or substituted phenyl wherein the substituents vary in number from 1 to 3 and are selected from chlorine, fluorine, bromine, iodine, trifluoromethyl, lower alkyl having from 1 to 4 carbon atoms and being straight or branched, lower alkoxy having from 1 to 4 carbon atoms and being straight or branched or $NR_5R_6$ wherein $R_5$ and $R_6$ have the meanings defined hereinabove; or

$$-\overset{\overset{O}{\|}}{C}Ph$$

wherein Ph is phenyl or phenyl substituted with from 1 to 3 substituents selected from chlorine, fluorine, bromine, iodine, trifluoromethyl, lower alkyl having from 1 to 4 carbon atoms and being straight or branched, lower alkoxy having from 1 to 4 carbon atoms and being straight or branched, or $NR_5R_6$ wherein $R_5$ and $R_6$ have the meanings defined hereinabove; or
(e) $R_3$ is hydrogen and $R_4$ is $NR_{12}R_{13}$ wherein each of $R_{12}$ and $R_{13}$ is selected from hydrogen, straight or branched alkyl having from 1 to 6 carbon atoms, phenyl or benzyl or phenyl or benzyl substituted on any positions 2 through 6 of the aromatic ring with from 1 to 3 substituents selected from fluorine, chlorine, bromine, iodine, trifluoromethyl, lower alkyl having from 1 to 4 carbon atoms and being straight or branched, lower alkoxy having from 1 to 4 carbon atoms and being straight or branched, or $NR_5R_6$ wherein $R_5$ and $R_6$ have the meanings defined hereinabove;
wherein each of $R_1$ and $R_2$ is independently

(a) hydrogen,
(b) the group

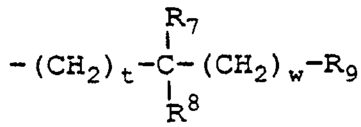

wherein t is zero to 4; w is zero to 4 with the proviso that the sum of t and w is not greater than 5; $R_7$ and $R_8$ are independently selected from hydrogen or alkyl having from 1 to 6 carbon atoms, or when $R_7$ is hydrogen, $R_8$ can be selected from the group defined for $R_9$; and $R_9$ is phenyl or phenyl substituted with from 1 to 3 substituents selected from straight or branched alkyl having from 1 to 6 carbon atoms, straight or branched alkoxy having from 1 to 6 carbon atoms, the group

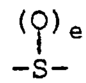

alkyl wherein e is zero, one or two, and alkyl is straight or branched and has from 1 to 6 carbon atoms, phenoxy, hydroxy, fluorine, chlorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above;
(c) the group

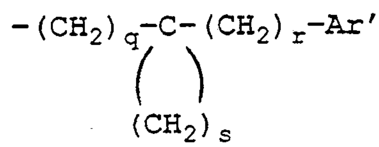

wherein q is zero to 3; r is zero to 2; s is 2 to 6; and Ar′ is
phenyl,
1- or 2-naphthyl,
phenyl or 1- or 2-naphthyl substituted with alkyl of from one to six carbon atoms,
alkoxy of from one to six carbon atoms,
hydroxy,
benzyloxy,
fluorine,
chlorine,
bromine,
nitro,
trifluoromethyl,
$-NH-COCH_3$,
$-CONH_2$,
-COOH,
-COOalkyl wherein alkyl has from one to four carbon atoms,
$-CH_2COOH$,
$-CH_2CONH_2$,
$-NR_5R_6$ wherein $R_5$ and $R_6$ have the meanings defined above;
(d) a straight or branched hydrocarbon chain having from 1 to 20 carbon atoms and which is saturated or contains from 1 to 3 double bonds;
(e) an alkyl group having from 1 to 6 carbon atoms wherein the terminal carbon is substituted with hydroxy, $-NR_5R_6$ wherein $R_5$ and $R_6$ have the meanings defined above, or -COOalkyl wherein alkyl is straight or branched and has from 1 to 4 carbon atoms;
(f) $-(CH_2)_pQ$ wherein p is a number from zero to 3 and Q is a 5- or 6-membered monocyclic or fused bicyclic heterocycle containing at least 1 to 4 nitrogen, oxygen, or sulfur atoms in at least one ring member;

(g) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, alkylthio which is straight or branched and has from 1 to 6 carbon atoms, $-NR_5R_6$ wherein $R_5$ and $R_6$ have the meanings defined above, hydroxy, nitro, chlorine, fluorine, bromine, or trifluoromethyl; or

(h) $NR_1R_2$ taken together are phenothiazine or form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, ω-hydroxyalkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro; or

a pharmaceutically acceptable salt thereof.

This invention also provides pharmaceutical compositions containing the compounds of Formula I and the use of the compounds for the preparation of pharmaceuticals useful for treating hypercholesterolemia and atherosclerosis. The compounds of Formula I wherein $R_1$ and $R_2$ are both hydrogen are useful as intermediates in preparing pharmaceutically active compounds of the invention. All the other compounds of Formula I are ACAT inhibitors.

## DETAILED DESCRIPTION OF INVENTION

The compounds of the present invention provide a novel class of diamides which are ACAT inhibitors rendering them useful in treating hypercholesterolemia and atherosclerosis.

Illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-ethyltetradecyl, and n-octadecyl groups.

Illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.

Straight or branched alkoxy groups having from 1 to 6 carbon atoms include, for example, methoxy, ethoxy, n-propoxy, t-butoxy, and pentyloxy.

The term alkylthio having from 1 to 6 carbon atoms means the group $C_{1-6}$alkyl-S- wherein the alkyl moiety is straight or branched.

A 5- or 6-membered monocyclic or fused bicyclic heterocycle is a monocyclic or fused bicyclic aromatic ring containing at least 1 to 4 heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, oxazolyl, isoxazolyl, triazolyl, tetrazolyl, imidazolyl, benzothiazolyl, indolyl, quinolinyl, isoquinolinyl, or N-oxides of heterocycle containing a nitrogen atom.

More specifically, such a heterocycle may be a 2-or 3-thienyl ; 2- or 3-furanyl; 2-, or 3-, or 4-pyridyl or -pyridyl-N-oxide; 2-, 4-, or 5-pyrimidinyl; 3- or 4-pyridazinyl; 2-pyrazinyl; 2-pyrazinyl-N-oxide; 2- or 3-pyrrolyl; 3-, 4-, or 5-pyrazolyl; 3-, 4-, or 5-osoxazolyl; 3-, 4-, or 5-isoxazolyl; 3-, 4-, or 5-oxazolyl; 3-, 4-, or 5-isothiazolyl; 5-tetrazolyl; 3- or 5-(1,2,4,-)triazolyl; 4- or 5-(1,2,3-)triazolyl; 2-, 4-, or 5-imidazolyl; 2-, 3-, 4-, 5-, 6-, or 7-indolyl; 2-, 3-, 4-, 5-, 6-, 7-, or 8-quinolinyl; 1-, 3-, 4-, 5-, 6-, 7-, or 8-isoquinolinyl; 2-, 4-, 5-, 6-, or 7-benzothiazolyl; or 2-, 3-, 4-, 5-, 6-, or 7-benzothienyl.

Preferred compounds of this invention are those wherein Ar is phenyl or Ar′ is phenyl or substituted phenyl and more preferably wherein Ar′ is phenyl substituted on the 2,6-positions. Other preferred compounds of this invention are those wherein $R_1$ or $R_2$ represents the group

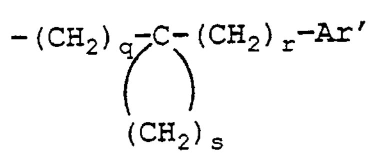

or the group

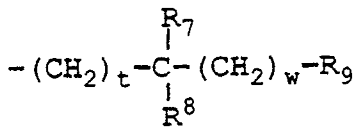

wherein q, r, s, Ar′, t, w, $R_7$, $R_8$, and $R_9$ have the meanings defined in Formula I.

Pharmaceutically acceptable salts of the compounds of Formula I are also included as a part of the present invention.

The acid salts may be generated from the free base by reaction of the latter with one equivalent of a suitable nontoxic, pharmaceutically acceptable acid, followed by evaporation of the solvent employed for the reaction and recrystallization of the salt if required. The free base may be recovered from the acid salt by reaction of the salt with an aqueous solution of a suitable base such as sodium carbonate, sodium bicarbonate, potassium carbonate, sodium hydroxide, and the like.

Suitable acids for forming acid salts of the compounds of this invention include, but are not necessarily limited to acetic, benzoic, benzenesulfonic, tartaric, hydrobromic, hydrochloric, citric, fumaric, gluconic, glucuronic, glutamic, lactic, malic, maleic, methanesulfonic, pamoic, salicylic, stearic, succinic, sulfuric, and tartaric acids. The class of acids suitable for the formation of nontoxic, pharmaceutically acceptable salts is well known to practitioners of the pharmaceutical formulation arts (see, for example, Stephen N. Berge, et al, J Pharm Sciences 66:1-19 (1977)).

The compounds of the present invention may also exist in different stereoisomeric forms by virtue of the presence of asymmetric centers in the compound. The present invention contemplates all stereoisomeric forms of the compounds as well as mixtures thereof, including racemic mixtures. Individual stereoisomers may be obtained, if desired, by methods known in the art as, for example, the separation of stereoisomers in chiral chromatographic columns.

Further, the compounds of this invention may exist in unsolvated as well as solvated forms with pharmaceutically acceptable solvents such as water, ethanol, and the like. In general, the solvated forms are considered equivalent to the unsolvated forms for the purposes of this invention.

As shown by the data presented below in Table 1, the compounds of the present invention are potent inhibitors of the enzyme acyl-CoA: cholesterol acyltransferase (ACAT), and are thus effective in inhibiting the esterification and transport of cholesterol across the intestinal cell wall. The compounds of the present invention are thus useful in pharmaceutical formulations for the treatment of hypercholesterolemia or atherosclerosis.

The ability of representative compounds of the present invention to inhibit ACAT was measured using an in vitro test more fully described in F. J. Field and R. G. Salone, Biochemica et Biophysica 712:557-570 (1982). The test assesses the ability of a test compound to inhibit the acylation of cholesterol by oleic acid by measuring the amount of radiolabeled cholesterol oleate formed from radiolabeled oleic acid in a tissue preparation containing rabbit intestinal microsomes.

The data appear in Table 1 where they are expressed as $IC_{50}$ values; i.e., the concentration of test compound required to inhibit the activity of the enzyme by 50%.

TABLE 1

| Compound | $IC_{50}$ (μM) |
|---|---|
| N-′2,6-bis(1-methylethyl)phenyl]-N′-(2,2-diphenylethyl)propanediamide | 0.77 |
| N-[2,6-bis(1-methylethyl)phenyl]-N′-(1-phenylcyclopentyl)methylpropanediamide | 0.99 |
| N′-′2,6-bis(1-methylethyl)phenyl]-N′-(diphenylmethyl)propanediamide | 0.49 |
| N′-[2,6-bis(1-methylethyl)phenyl]-N,N-bis(phenylmethyl)propanediamide | 0.013 |
| N′-[2,6-bis(1-methylethyl)phenyl]-N-(diphenylmethyl)-N-(1-methylethyl)propanediamide | 0.020 |

In one in vivo screen designated APCC, male Sprague-Dawley rats (200 to 225 g) were randomly divided into treatment groups and dosed at 4 PM with either vehicle (CMC/Tween) or suspensions of compounds in vehicle. The normal chow diet was then replaced with the PCC diet with either 1% or 0.5%

cholic acid, as indicated. The rats consumed this diet ad libitum during the night and were sacrificed at 8 AM to obtain blood samples for cholesterol analysis using standard procedures. Statistical differences between mean cholesterol values for the same vehicle were determined using analysis of variance followed by Fisher's least significant test. The results of this trial for representative compounds of the present invention appear in Table 2.

TABLE 2

| Compound of Example | % Change (mg/dl) |
|---|---|
| N-′2,6-bis(1-methylethyl)phenyl]-N′-(1-phenylcyclopentyl)methylpropanediamide | +7 |
| N-[2,6-bis(1-methylethyl)phenyl]-N′-(diphenylmethyl)propanediamide | -20 |
| N-[2,6-bis(1-methylethyl)phenyl]N,N-bis(phenylmethyl)propanediamide | -42 |
| N′-[2,6-bis(1-methylethyl)phenyl]-N-(diphenylmethyl)-N-(1-methylethyl)propanediamide | -41 |

In therapeutic use as agents for treating hypercholesterolemia or atherosclerosis, the compounds of Formula I or pharmaceutically acceptable salts thereof are administered to the patient at dosage levels of from 250 to 3000 mg per day. For a normal human adult of approximately 70 kg of body weight, this translates into a dosage of from 5 to 40 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing the pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active component is mixed with the carrier having the necessary binding properties in suitable proportions and compacted in the shape and size desired.

Powders and tablets preferably contain between about 5% to about 70% by weight of the active ingredient. Suitable carriers are magnesium dicarbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner cachets are also included.

Tablets, powders, cachets, and capsules can be used as solid dosage forms suitable for oral administration.

Liquid form preparations include solutions, suspensions, or emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethylcellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation containing discrete quantities of the preparation, for example, packeted tablets, capsules, and powders in vials or ampoules. The unit dosage form can also be a capsule, cachet, or tablet itself, or it can be the appropriate number of these packaged forms.

The compounds of this invention may be prepared by various means, all of which involve generally well-known procedures. In Chart I, there is set forth a reaction scheme for preparing the compounds of Formula I wherein $R_4$ is other than the group $-NHR_{10}$ or $NR_{12}R_{13}$. An arylamine (1) is reacted with an

appropriate acid halide, e.g., the acid chloride (2) in an aprotic, nonpolar solvent such as ethyl acetate, tetrahydrofuran, diethyl ether, or methylene chloride in the presence of a tertiary amine such as triethylamine, diisopropyl ethylamine or pyridine for about 1 to 24 hours at a temperature of from about 0° to 25° C to give the amide (3). The amide (3) is converted to the free acid (4) by treatment with a mild aqueous base, e.g., sodium hydroxide in a lower alcohol such as methanol or ethanol at a temperature of from 25°-80° C for from about 1 to 20 hours. The free acid can be converted to the bis-amide (5) by various means and essentially by any means which affords an activated ester to which is added an appropriate amine of the formula $NHR_1R_2$ wherein $R_1$ and $R_2$ have the meanings defined in Formula I. For example, the acid (4) can be treated with a carbodiimide such as dicyclohexyl carbodiimide in methylene chloride and reacted with the amine at a temperature varying from about 0° to 25° C. Alternatively, the acid (4) can be treated with a chloroformate such as methyl, ethyl, or isobutyl chloroformate in the presence of a tertiary amine, e.g., triethylamine, to give a mixed anhydride which is then reacted with an amine of the formula $NHR_1R_2$. Additionally, the acid (4) can be treated with carbonyldiimidazole and reacted with an appropriate amine or the acid (4) can be converted to the corresponding acid chloride by treatment with thionyl chloride or oxalyl chloride/dimethylformamide, and reacted with an appropriate amine of the formula $NHR_1R_2$ in the presence of base. The amines, $NHR_1R_2$, are commercially available or are prepared by procedures well known in the art.

The compounds of this invention wherein $R_4$ is other than the group $-NHR_{10}$ can also be prepared as set forth in Chart II whereby an appropriate aryl amine (1) is reacted with an acid anhydride (6) in an aprotic nonpolar solvent to give the acid (4) which is then converted to the bis-amide as described above. It should be recognized that in the scheme of Chart II the addition of the amine groups could be reversed. That is, the acid anhydride could first be reacted with the amine $NHR_1R_2$ to give a compound of formula (7) set forth in Chart I which could then be converted to compounds (5) by treating compounds (7) in a manner as described above to afford an activated ester to which is added an appropriate aryl amine of the formula $ArNH_2$.

Further, the compounds of Formula I wherein $R_3$ and $R_4$ are other than hydrogen can be prepared from the corresponding compounds wherein $R_3$ and $R_4$ are hydrogen by treatment with a mild base such as sodium hydride or sodium carbonate/dimethylformamide and alkylating with an appropriate alkylhalide of the formula $R_3$halide or $R_4$halide wherein $R_3$ and $R_4$ have the same meaning as defined in Formula I but are not hydrogen, and halide is a halogen-atom, e.g., chloride.

The compounds of this invention wherein $R_3$ is hydrogen and $R_4$ is the group $-NHR_{10}$ or $-NR_{12}R_{13}$ are prepared by reacting a compound of the formula

$$R_{11}OOC-(CH_2)_m-\underset{}{\overset{NHPr}{\overset{|}{C}}}H-COOH \qquad (A)$$

wherein $R^{11}$ is a lower alkyl $C_{1-4}$, m is zero, one or two and Pr is a protecting group such as the tert-butyloxycarbonyl or benzyloxycarbonyl with a carbodiimide such as dicyclohexylcarbodiimide followed by addition of an amine of the formula $NHR_1R_2$ wherein $R_1$ and $R_2$ have the meanings defined hereinabove at a temperature varying from about 0° C to 25° C. The resulting amide ester

$$R_{11}OOC-(CH_2)_m-\overset{NHPr}{\overset{|}{C}}HCONR_1R_2 \qquad (B)$$

is then converted to the amide acid by mild base hydrolysis to give compounds of the formula

$$HOOC-(CH_2)_m-\overset{NHPr}{\overset{|}{C}}H-CONR_1R_2 \qquad (C)$$

which are treated with a carbodiimide as generally described above followed by addition of an appropriate aryl amine of the formula $ArNH_2$ wherein Ar has the meaning defined above to give compounds

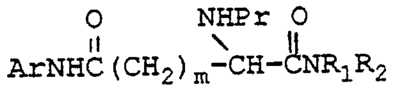

(D)

If desired, the protecting group can be removed to give compounds of Formula I wherein $R_3$ is hydrogen and $R_4$ is the group $-NR_{12}R_{13}$ wherein $R_{12}$ and $R_{13}$ are hydrogen. The latter compounds can be acylated by generally known procedures, e.g., by reaction with an appropriate acid chloride or isocyanate or thioisocyanate to give compounds of Formula I wherein $R_3$ is hydrogen and $R_4$ is $NHR_{10}$ or the latter compounds can be alkylated by procedures generally known in the art to give compounds of Formula I wherein $R_3$ is hydrogen and $R_4$ is $NR_{12}R_{13}$ wherein $R_{12}$ and $R_{13}$ are both not hydrogen.

It is apparent from the foregoing that compounds of the following formula (E) wherein n is zero, one or two can be obtained by following the above general procedure, only reversing the order of the amine additions.

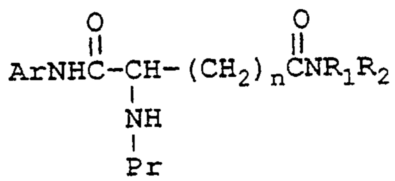

(E)

The compounds of Formula (A) are commercially available or are obtained from a corresponding diester derivative.

EXAMPLE 1

N-[2,6-Bis(1-methylethyl)phenyl]-N′-(2,2-diphenylethyl)propanediamide

(a) To a mixture of 43.30 g (0.24 mole) of 2,6-diisopropylaniline and excess triethylamine in 400 mL of ethyl acetate was added dropwise 36.7 g (0.24 mole) of ethyl malonylchloride (technical) at 0 °C; more ethyl acetate was added as the reaction mixture became a slurry. The reaction mixture was stirred for 1 hour at 0 °C then allowed to sit at room temperature for 30 minutes. The resulting white precipitate was filtered off and concentrated on a rotary evaporator, leaving an oil which crystallized. Hexane was added to the crystalline material and the solid collected by filtration to give 3-[[2,6-bis(1-methylethyl)phenyl]amino]-3-oxopropionic acid ethyl ester.

(b) To 4.6 g (0.016 mole) of the ethyl ester obtained above in 50 mL MeOH was added 17 mL 1M NaOH at room temperature, after which water was added until the solution became cloudy. The solution was allowed to sit at room temperature for 2 hours then concentrated on a rotary evaporator, diluted with $H_2O$ and EtOAc and made acidic with 1N HCl. Brine was added and the layers were separated, the organic layer was washed with brine, dried over $MgSO_4$, filtered, and stripped to a white flaky solid. The solid was recrystallized from hexane to give 3-[[2-bis(2-methyl)phenyl]amino]-3-oxopropionic acid.

(c) To a mixture of 0.52 g (0.002 mole) of the above-obtained acid and 0.40 g (0.002 mole) of 2,2-diphenylethylamine in 25 mL $CH_2Cl_2$ was added 0.41 g (0.002 mole) of dicyclohexylcarbodiimide at room temperature. The reaction mixture was allowed to stand 4 days at room temperature, then was diluted with EtOAc, washed with $H_3PO_4$ and brine, dried over $MgSO_4$, filtered, and concentrated to a viscous oil/glass. The concentrate was recrystallized from ethyl acetate and ethyl acetate/hexane (50:50) to give the title compound.

IR-IB 890914; MS-MB 891126; NBR-ND 893682.

| | | | |
|---|---|---|---|
| Calc.: | C, 78.70; | H, 7.74; | N, 6.33. |
| Found: | C, 78.42; | H, 7.74; | N, 6.30. |

When in the procedure of Example 1(c) an appropriate amount of the amine set forth in the following Table 3 is substituted for 2,2-diphenylethylamine, the respective product listed in Table 3 is obtained:

TABLE 3

| Amine | Product |
|---|---|
| 1-phenylcyclopentyl-methyl amine | N-[2,6-bis(1-methylethyl)-phenyl]-N'-(1-phenylcyclopentyl)methylpropanediamide; IR (KBr) 3278, 2962, 1670, 1653, 1628, 1497 $cm^{-1}$. |
| diphenylmethylamine | N-[2,6-bis(1-methylethyl)-phenyl-N'-(diphenylmethyl)-propanediamide; IR (KBr) 3262, 1664, 1644, 1521, 699 $cm^{-1}$. |
| N,N-di(phenylmethyl) amine | N'-[2,6-bis(1-methylethyl)-phenyl]-N,N-bis(phenylmethyl)-propanediamide; IR (KBr) 3224, 2962, 1674, 1614, 1533, 1449, 703 $cm^{-1}$. |
| N-diphenylmethyl-N-(1-methylethyl)amine | N'-[2,6-bis(1-methylethyl)-phenyl]-N-(diphenylmethyl)-N-(1-methylethyl)propanediamide; IR (KBr) 3280, 2964, 1676, 1627, 708 $cm^{-1}$. |
| N-phenylpiperazino | N-[2,6-bis(1-methylethyl)-phenyl]-ß-oxo-4-phenyl-1-piperazinepropanamide; IR (KBr) 3216, 2961, 1684, 1628, 1506, 1459, 1230, 758 $cm^{-1}$. |
| 4-phenylpiperidino | N-[2,6-bis(1-methylethyl)-phenyl]-ß-oxo-4-phenyl-1-piperidinepropanamide; IR (KBr) 2965, 1660, 1645, 1496, 1445, 1214, 699 $cm^{-1}$. |
| 2-hydroxymethyl-pyrrolidino | (S)-N-[2,6-bis(1-methylethyl)phenyl]-2-(hydroxymethyl)-ß-oxo-1-pyrrolidine-propanamide; IR (KBr) 3371, 3248, 1640, 1534, 1451, 1067 $cm^{-1}$. |
| N-phenylmethyl-N-(1-methylethyl)amino | N'-[2,6-bis(1-methylethyl)-phenyl]-N-(1-methylethyl)-N-(phenylmethyl)propanediamide; IR (KBr) 3245, 2961, 1682, 1618, 1607, 1533, 1449, 716 $cm^{-1}$. |

TABLE 3

| Amine | Product |
|---|---|
| N-(2-pyridylethyl)-N-methylamine | N′-[2,6-bis(1-methylethyl)-phenyl]-N-methyl-N-[2-(2-pyridyl)ethyl]propanediamide; IR (KBr) 3294, 2963, 1666, 1652, 1644, 1509, 1434, 748 $cm^{-1}$. |
| N,N-dipentylamine | N,N-dipentyl-N′-[2,6-bis(1-methylethyl)phenyl]propane-diamide |
| phenylmethylamine | N-[2,6-bis(1-methylethyl)-phenyl]-N′-(phenylmethyl)-propanediamide; IR (KBr) 3316, 3234, 1650, 1546, 1528, 1256, 746 $cm^{-1}$. |
| N,N-di-(2-hydroxy-ethyl)amine | N,N-di-(2-hydroxyethyl)-N′-[2,6-bis(1-methylethyl)-phenyl]propanediamide; IR (KBr) 3410, 3252, 3245, 1655, 1634, 1363, 624 $cm^{-1}$. |
| N-(4-fluorophenyl-methyl)-N-(1-methyl-ethyl)amine | N′-[2,6-bis(1-methylethyl)-phenyl]-N-[(4-fluorophenyl)-methyl]-N-(1-methylethyl)-propanediamide; IR (KBr) 3293, 1671, 1630, 1512, 1223, 797 $cm^{-1}$. |
| N-(2-dimethylamino-ethyl)-N-phenyl-methylamine | N-[2,6-bis(1-methylethyl)-phenyl]-N′-(2-dimethyl-aminoethyl)-N′-(phenyl-methyl)propanediamide; IR (KBr) 3185, 2964, 1623, 1609, 1531, 1459, 703 $cm^{-1}$. |
| N-(2-hydroxyethyl-N-phenylmethylamine | N-[2,6-bis(1-methylethyl)-phenyl]-N′-(2-hydroxyethyl)-N′-(phenylmethyl)propane-diamide; IR (KBr) 3249, 2964, 1654, 1627, 1533, 700 $cm^{-1}$. |
| N-ethyl-N-(2-diethylaminoethyl)-amine | N-[2,6-bis(1-methylethyl)-phenyl]-N′-(ethyl)-N′-(2-diethylaminoethyl)propane-diamide; IR (Q film) 3243, 2937, 2931, 1685, 1670, 1624, 1203, 785 $cm^{-1}$. |

TABLE 3

| Amine | Product |
|---|---|
| N-(2-hydroxyethyl)-piperazine | N-[2,6-bis(1-methylethyl)-phenyl]-ß-oxo-4-(2-hydroxy-ethyl)-1-piperazine-propanamide; IR (KBr) 3426, 2961, 1638, 1525, 1054, 733 $cm^{-1}$. |
| morpholine | N-[2,6-bis(1-methylethyl)-phenyl]-ß-oxo-1-morpholine-propanamide; IR (KBr) 3235, 1669, 1529, 1440, 1224, 798 $cm^{-1}$. |
| thiomorpholine | N-[2,6-bis(1-methylethyl)-phenyl]-ß-oxo-1-thiomor-pholinepropanamide; IR (KBr) 3439, 1654, 1467, 1343, 961, 796 $cm^{-1}$. |
| N-(2-cyanoethyl)-N-(phenylmethyl)amine | N-[2,6-bis(1-methylethyl)-phenyl]-N'-[2-cyanoethyl)-N'-(phenylmethyl)propanediamide; IR (KBr) 3259, 2969, 1642, 1533, 1335, 742 $cm^{-1}$. |
| isopropylphenyl-amine | N'-[2,6-bis(1-methylethyl)-phenyl]-N-(1-methylethyl)-N-phenylpropanediamide; IR (KBr) 3261, 2966, 1645, 1629, 1538, 1531, 1522, 1353, 703 $cm^{-1}$. |
| isopropyl-2-pyridyl-methylamine | N-[2,6-bis(1-methylethyl)-phenyl]-N-(1-methylethyl)-N-[(3-pyridinyl)methyl]propane-diamide; IR (KBr) 2967, 1666, 1630, 1539, 1413, 971, 713 $cm^{-1}$. |
| N-benzylpiperazine | N-[2,6-bis(1-methylethyl)-phenyl]-ß-oxo-4-(phenyl-methyl)-1-piperazine-propanamide; IR (KBr) 3263, 2961, 1684, 1618, 1521, 1457, 1000, 699 $cm^{-1}$. |
| diphenylamine | N'-[2,6-bis(1-methylethyl)-phenyl]-N,N-diphenylpropane-diamide; IR KBR 3243, 1661, 1646, 1540, 1349, 695 $cm^{-1}$. |

TABLE 3

| Amine | Product |
|---|---|
| isopropyl-4-methyl-thiophenylamine | N'-[2,6-bis(1-methylethyl)-phenyl]-N-(1-methylethyl)-N-[[4-(methylthio)phenyl]-methyl]propanediamide; IR (KBr) 3248, 2965, 1684, 1601, 1331, 799 $cm^{-1}$. |
| phenylaminoacetic acid, ethyl ester | N-[3-[[2,6-bis(1-methylethyl)-phenyl]amino]-1,3-dioxopropyl]-N-phenylglycine, ethyl ester; IR (KBr) 3238, 1738, 1646, 1389, 1208, 763 $cm^{-1}$. |
| N-(2-methoxyphenyl)-piperazine | N-[2,6-bis(1,1-dimethylethyl)-phenyl]-4-(2-methoxyphenyl)-ß-oxo-1-piperazinepropanamide; IR (KBr) 3265, 1654, 1629, 1489, 1040, 935 $cm^{-1}$. |
| N-benzhydryl-piperazine | N-[2,6-bis(1-methylethyl)-phenyl]-1-(diphenylmethyl)-1-piperazinepropanamide; IR (KBr) 3391, 3291, 2963, 1634, 1452, 796, 706 $cm^{-1}$. |
| 4-methoxybenzyl-2-pyridylamine | N-[2,6-bis(1-methylethyl)-phenyl]-ß-[[(4-methoxyphenyl)-methyl](2-pyridinyl)amino]-ß-oxopropanamide; IR (KBr) 3254, 3248, 2931, 1654, 1467, 1250, 799 $cm^{-1}$. |
| phenothiazine | N-[2,6-bis(1-methylethyl)-phenyl]-ß-oxo-10H-phenothiazine-10-propanamide; IR (KBr) 3330, 2963, 1654, 1474, 1344, 739 $cm^{-1}$. |
| 4-methoxyphenyl-2-pyrimidinylamine | N'-[2,6-bis(1-methylethyl)-phenyl]-N-[(4-methoxyphenyl)-methyl]-N-(2-pyrimidinyl)-propanediamide; IR (KBr) 3298, 3238, 2962, 1669, 1514, 1414, 808 $cm^{-1}$. |
| heptylamine | N-[2,6-bis(1-methylethyl)-phenyl]-N'-heptylpropane-diamide; IR (KBr) 3327, 3273, 1671, 1523, 1443, 748 $cm^{-1}$. |

TABLE 3

| Amine | Product |
|---|---|
| 2,6-diisopropyl-phenylamine | N,N'-bis[2,6-bis(1-methyl-ethyl)phenyl]propanediamide; IR (KBr) 3233, 1648, 1520, 1257, 715 $cm^{-1}$. |
| (4-dimethylamino-benzyl)(isopropyl)-amine | N'-[2,6-bis(1-methylethyl)-phenyl]-N-[[4-(dimethylamino)-phenyl]methyl]-N-(1-methyl-ethyl)propanediamide; IR (KBr) 3262, 2963, 1617, 1525, 1447, 1346, 1069, 743 $cm^{-1}$. |
| 1,1,3,3-tetramethyl-butylamine | N-[2,6-bis(1-methylethyl)-phenyl]-N'-(1,1,3,3-tetra-methylbutyl)propanediamide; IR (KBr) 3438, 3297, 2936, 1653, 1540, 1473, 1230 $cm^{-1}$. |
| (methyl(octyl)amine | N'-[2,6-bis(1-methylethyl)-phenyl]-N-methyl-N-octyl-propanediamide; IR (KBr) 3284, 3263, 2927, 1654, 1508, 1129, 717 $cm^{-1}$. |
| 1,1-dimethyl-2-phenylethylamine | N-[2,6-bis(1-methylethyl)-phenyl]-N'-(1,1-dimethyl-2-phenylethyl)propanediamide; IR (KBr) 3297, 2964, 1643, 1559, 1457, 1364, 702 $cm^{-1}$. |
| 2,6-bis(dimethyl-amino)benzylamine | N-[2,6-bis(dimethylamino)-phenyl]-2,6-bis(1-methyl-ethyl)phenyl]propanediamide; IR (KBr) 3462, 3243, 1658, 1507, 1324, 727 $cm^{-1}$. |
| (methyl)(octadecyl)-amine | N'-[2,6-bis(1-methylethyl)-phenyl]-N-methyl-N-octadecyl-propanediamide; IR (KBr) 3297, 2961, 2923, 1653, 1629, 1512, 1406, 719 $cm^{-1}$. |

Following the general procedure of Example 6, only substituting an appropriate amount of pyridine-3-carboxaldehyde or 4-dimethylaminobenzaldehyde for 4-thiomethylbenzaldehyde, the following respective amines were obtained: N-(1-methylethyl)-3-pyridinemethanamine and 4-(dimethylamino)-N-(1-methylethyl)-benzenemethanamine.

EXAMPLE 2

N-(1-Methylethyl)diphenylmethylamine

A mixture of 9.88 g (0.04 mole) of benzhydryl bromide, 2.4 g (0.04 mole) of isopropyl amine, and excess triethylamine in 300 mL of ethyl acetate was stirred overnight at room temperature. The reaction mixture was concentrated in vacuo, after which more triethylamine was added and the reaction mixture was heated on a steambath. Excess concentrated HCl was added, then the mixture was washed with ethyl acetate, made basic with sodium hydroxide, extracted with ethyl acetate, dried over magnesium sulfate, filtered, and concentrated in vacuo to give the title compound as an oil.

EXAMPLE 3

N-[2,6-Bis(1-methylethyl)phenyl]-N′-(diphenylmethyl)pentanediamide

(a) A mixture of 3.34 g (0.02 mole) 2,6-diisopropyl aniline and 2.31 g (0.02 mole) glutaric anhydride in 50 mL EtOAc was heated on the steambath overnight then allowed to sit 3 days and nights at room temperature. The mixture was taken up in EtOAc, washed with 1N HCl and brine, dried over $MgSO_4$, filtered, and stripped to a viscous oil. Addition of 1/1 hexane/EtOAc caused precipitation of white crystals to give 4-[2,6-bis(1-methylethyl)phenylaminocarbonylobutyric acid.

(b) To a mixture of 0.58 g (0.002 mole) of the above obtained acid, 0.40 g (0.0022 mole) benzhydryl amine, and 100 mL $CH_2Cl_2$ was added all at once 0.44 g (0.0021 mole) of dicyclohexylcarbodiimide at 0 °C. Stirring was continued and the reaction mixture was allowed to warm to room temperature overnight. The reaction mixture was allowed to sit 2 days at room temperature, filtered, and concentrated in vacuo. The concentrate was taken up in $CH_2Cl_2$ and filtered through $SiO_2$ (70-250) using $CH_2Cl_2$ as eluant to give the title compound. IR (KBr) 3262, 1655, 1638, 1524, 697 $cm^{-1}$.

EXAMPLE 4

4-[2,2-Diphenylethyl)aminocarbonyl]butyric acid

A mixture of 0.99 g (0.005 mole) 2,2-diphenylethylamine, 0.58 g (0.005 mole) glutaric anhydride, and 50 mL EtOAc was heated on the steambath until solution occurred. The solution was concentrated in vacuo to an oil, which was filtered through $SiO_2$ (70-250) using EtOAc as eluant to give the title compound.

When in the procedure of Example 4 an appropriate amount of benzhydrylamine or dibenzylamine is substituted for 2,2-diphenylethylamine, the following respective acids were obtained:
4-[[(diphenylmethyl)amino]carbonyl]butyric acid.
4-[[N,N-bis(phenylmethyl)amino]carbonyl]butyric acid.

EXAMPLE 5

3-[2(2,2-Diphenylethyl)aminocarbonyl]propionic acid

A mixture of 0.99 g (0.005 mole) 2,2-diphenylethylamine and 0.50 g (0.005 mole) succinic anhydride in 50 mL EtOAc was heated until solution occurred. The solution was allowed to sit overnight at room temperature, then concentrated in vacuo. The concentrate was taken up in EtOAc and hexane, and the resulting solid collected by filtration. The solid was washed with EtOAc to give the title compound.

When in the procedure of Example 5 an appropriate amount of diphenylmethylamine or dibenzylamine

is substituted for 2,2-diphenylethylamine the following acids were obtained:
3-[(diphenylmethyl)aminocarbonyl]propionic acid.
3-[[N,N-bis(phenylmethyl)amino]carbonyl]propionic acid.

EXAMPLE 6

N-(1-Methylethyl)-4-(methylthio)benzenemethanamine

4-Thiomethylbenzaldehyde (15.3 g, 100 mmole) and isopropylamine (8.0 g, 140 mmole) were mixed together in 200 mL methanol with about 40 g of calcium sulfate as the drying agent. The mixture was stirred overnight at room temperature after which 3.9 g of sodium borohydride was added over one-half hour. The reaction mixture was stirred overnight at room temperature then filtered through filter cell. The filtrate was concentrated to dryness, and the resulting residue was partitioned between 150 mL 1N sodium hydroxide and ethyl acetate. The organic layer was washed with saturated aqueous sodium chloride, dried over magnesium sulfate, filtered and stripped to a clean oil to give the title compound. IR (KBr) 3248, 1684, 1601, 1522, 1331, 799 $cm^{-1}$.

EXAMPLE 7

N′-[2,6-Bis(1-methylethyl)phenyl]-N-(1-methylethyl)-N-[[4-(methylsulfinyl)phenyl]methyl]propanediamide

N′-[2,6-Bis(1-methylethyl)phenyl]-N-(1-methylethyl)-N-[[4-(methylthio)phenyl]methyl]propanediamide (1.40 g, 3.2 mmole) and metachloroperbenzoic acid (0.69 g, 80%) were stirred overnight at room temperature. The reaction mixture was concentrated to dryness, taken up in ethyl acetate, washed with aqueous $NaHSO_3$, aqueous $K_2CO_3$, and aqueous NaCl, dried ($MgSO_4$), and concentrated. The product was purified by filtration through $SiO_2$ (70-230 mesh) using ethyl acetate as eluant. The product was obtained as a white foam. 0.51 g, 35%. IR (KBr) 3253, 2965, 1677, 1637, 1521, 799 $cm^{-1}$.

EXAMPLE 8

N′-[2,6-Bis(1-methylethyl)phenyl]-2,2-dimethyl-N-(1-methylethyl)-N-(phenylmethyl)propanediamide

Oxalyl chloride (50 mL) was added to 5.0 g dimethylmalonic acid and a few drops DMF at room temperature. Reaction was immediate. The excess oxalyl chloride was removed on the rotary evaporator, leaving behind a mobile yellow oil. This oil was added to a mixture of 6.7 g 2,6-diisopropylaniline, 5.6 g N-benzyl isopropylamine, and excess triethylamine in 250 mL ethyl acetate at room temperature. The reaction mixture was concentrated to dryness after sitting 30 minutes at room temperature. The residue was taken up in ethyl acetate and concentrated again on the rotary evaporator. The residue was taken up in ethyl acetate, washed with 200 mL 1N HCl and brine, dried over $MgSO_4$, filtered, and concentrated to a brown oil. Attempted crystallization from hexane and 1/1 hexane/EtOAc gave only impure product. The brown oil was then filtered through silica gel (70-230 mesh) using 1/1 hexane/EtOAc as eluant to give the product as an oil. The oil was crystallized from diethyl ether. 1.66 g, 10.4%. IR (KBr) 3335, 2967, 1670, 1624, 1497 $cm^{-1}$.

EXAMPLE 9

(±) [1-[[[2,6-Bis(1-methylethyl)phenyl]amino]carbonyl]-2-[(1-methylethyl)(phenylmethyl)amino]-2-oxoethyl]-carbamic acid, 1,1-dimethylethyl ester

(a) A solution of 31.7 g (150 mmole) of diethyl aminomalonate hydrochloride in 400 mL 1/1 $THF/H_2O$ was cooled to 0° C and 20 mL triethylamine was added followed by 35 g di-t-butyldicarbonate. The reaction mixture was stirred 1.25 hours at 0° C and was then warmed briefly to 50° C. The reaction mixture was stirred overnight at room temperature, concentrated to remove THF, diluted with ethyl acetate, washed with 200 mL 10% citric acid solution and brine, dried ($MgSO_4$), filtered, and concentrated to a clear viscous

liquid to give N-[bis(ethoxycarbonyl)methyl]carbamic acid, 1,1-dimethylethyl ester. 43.3 g, 100%. IR (LF) 2938, 1761, 1720, 1506, 1370, 1163, 780 $cm^{-1}$.

(b) The ester obtained in (a) above (11.02 g, 40 mmole) in 30 mL ethanol was mixed with 40 mL 1N NaOH and then heated gently (40° C) while slowly removing the solvent on the rotary evaporator. Water was added and the mixture was concentrated to a viscous oil. The oil was taken up in $H_2O$ and allowed to sit overnight at room temperature. The aqueous solution was diluted with $H_2O$, made acidic with 10% citric acid solution, and extracted twice with diethyl ether. The ether solution was dried ($MgSO_4$), filtered, and concentrated to give the N-[(carboxy)(ethoxycarbonyl)methyl]carbamic acid, 1,1-dimethylethyl ester as an oil (9.19 g, 92.8%), which crystallized on standing. IR (KBr) 3288, 2985, 1767, 1757, 1665, 1656, 1369, 1169, 777 $cm^{-1}$.

(c) A mixture of the ester from (b) above (9.0 g, 36.4 mmole) and 2,6-diisopropylaniline (7.0 g) in 400 mL methylene chloride was cooled to 0° C and 8.0 g dicyclohexylcarbodiimide was added all at once. The reaction mixture was stirred at 0° C and was allowed to slowly warm to room temperature and was then stirred overnight at room temperature. The reaction mixture was refluxed gently for 2 hours and then concentrated to dryness. The residue was taken up in ethyl acetate, washed with 10% citric acid solution, $NaHCO_3$ (aq) and NaCl (aq). The organic layer was dried ($MgSO_4$), filtered, and concentrated to an off-white solid which was slurried in hexane and collected by filtration to give 3-[[2,6-bis(1-methylethyl)phenyl]amino]-2-[[(1,1-dimethylethoxy)carbonyl]amino]-3-oxopropanoic acid, ethyl ester, 9.51 g (64%). IR (KBr) 3267, 2966, 1666, 1528, 1311, 735 $cm^{-1}$.

(d) The ester from (c) above (7.7 g, 19 mmole) was taken up in 50 mL methanol and 25 mL 1N NaOH was added at room temperature. The reaction mixture was concentrated on the rotary, water added, 5 mL more 1N NaOH added, and 10 mL methanol added. The mixture was heated briefly (~5 minutes) on the steambath, then concentrated on the rotary evaporator to remove methanol, diluted with water and washed with ethyl acetate. The aqueous layer was made acidic with citric acid solution, saturated with NaCl(s), and extracted with ethyl acetate. The ethyl acetate solution was dried ($MgSO_4$), filtered, and concentrated to give 3-[[2,6-bis(1-methylethyl)phenyl]amino]-2-[[(1,1-dimethylethoxy)carbonyl]amino]-3-oxopropanoic acid as a white solid, 5.69 g, 79.5%. IR (KBr) 3307, 2968, 1692, 1661, 1507, 1166, 744 $cm^{-1}$.

(e) Carbonyl diimidazole (2.5 g) was added to a mixture of 5.0 g (13.2 mmole) of the acid obtained in (d) above and 200 mL $CH_2Cl_2$ at room temperature. The resulting yellow solution was warmed to 30° C for ~5 minutes and then 2.5 g N-benzyl isopropylamine was added. The reaction mixture was stirred overnight at room temperature, concentrated to dryness, and the residue taken up in ethyl acetate. The solution was washed with $H_2O$, 10% citric acid solution, $NaHCO_3$ (aq), and brine, dried ($MgSO_4$), filtered, and concentrated to a yellow oil. The oil was purified by filtration through silica gel (70-230 mesh) using 8/2 hexane/EtOAc as eluant. The title compound was obtained as an oil/foam, 4.96 g (73.7%). IR (Kbr) 3278, 1729, 1669, 1499, 1364, 1282, 781 $cm^{-1}$.

## EXAMPLE 10

2-Amino-N′-[2,6-bis(1-methylethyl)phenyl]-N-(1-methylethyl)-N-(phenylmethyl) propanediamide

Trifluoroacetic acid (15 mL) was added to the product of Example 9 (2.47 g, 4.8 mmole) at room temperature. The reaction mixture was allowed to sit at room temperature over the weekend (2 days). The reaction mixture was concentrated to a viscous oil which was partitioned between $NaHCO_3$ (aq) and ethyl acetate. The ethyl acetate solution was washed with NaCl (aq), dried ($MgSO_4$), filtered, and concentrated to give the title compound as an off-white foam, 2.81 g (94.3%) . IR (Kbr) 3297, 2965, 1646, 1496, 925, 729 $cm^{-1}$.

## EXAMPLE 11

N′-[2,6-Bis(1-methylethyl)phenyl]-N-(1-methylethyl)-N-(phenylmethyl)-2-[[(propylamino)carbonyl]amino]-propanediamide

Propyl isocyanate (0.5 mL) was added to the product of Example 10 (0.36 g, 0.88 mmole) in 15 mL ethyl acetate at room temperature. The reaction mixture was concentrated on the rotary evaporator and the residue was treated a second time with .5 mL propyl isocyanate in ethyl acetate. The volatiles were removed in vacuo and the residue was allowed to sit overnight at room temperature. Addition of

hexane/EtOAc 1/1 caused precipitation of a white solid which was collected by filtration to give 0.32 g (75%) of the title compound. IR (Kbr) 3362, 1631, 1466, 732 $cm^{-1}$.

EXAMPLE 12

N′-[2,6-Bis(1-methylethyl)phenyl]-2-[(4-fluorobenzoyl)amino]-N-(1-methylethyl)-N-(phenylmethyl)-propanediamide

A mixture of the product of Example 10 (0.33 g, 0.81 mmole) and excess triethylamine in ethyl acetate was treated with 0.17 g 4-F benzoyl chloride at room temperature. The reaction mixture was allowed to sit 5 days at room temperature. The reaction mixture was then diluted with ethyl acetate, washed with $Na_2CO_3$ -(aq), 1N HCl, NaCl (aq), dried ($MgSO_4$), filtered, and concentrated to give the title compound as a white solid, 0.38 g (88.4%) . IR (Kbr) 3272, 2967, 1625, 1489, 1237, 768 $cm^{-1}$.

EXAMPLE 13

N′-[2,6-Bis(1-methylethyl)phenyl]-2-[[[(2-methoxyphenyl)amino]carbonyl]amino]-N-(1-methylethyl)-N-(phenylmethyl) propanediamide

A solution of the product of Example 10 (0.35 g, 0.85 mmole) in 15 mL ethyl acetate was treated with 0.14 g 2-methoxyphenylisocyanate. The reaction mixture was concentrated to a viscous oil. The oil was dissolved in ethyl acetate and allowed to site 5 days at room temperature. A white solid was formed and upon the addition of hexane was collected by filtration to give the title compound, 0.33 g (70%). IR (Kbr) 3374, 1686, 1546, 1516, 1443, 743 $cm^{-1}$.

EXAMPLE 14

N′-[2,6-Bis(1-methylethyl)phenyl]-2-[[[[4-(dimethylamino)phenyl]amino]thioxomethyl]amino]-N-(1-methylethyl)-N-(phenylmethyl)propanediamide

A mixture of the product of Example 10 (0.32 g, 0.78 mmole) and 0.16 g 4-dimethylaminophenyl isothiocyanate were allowed to sit 8 days at room temperature in ethyl acetate. The reaction mixture was concentrated to a viscous oil, which crystallized upon the addition of 9/1 hexane/ethyl acetate. The product was collected by filtration to give the title compound, 0.40 g (87%). IR (Kbr) 3316, 3199, 1515, 1362, 1173, 732 $cm^{-1}$.

CHART I

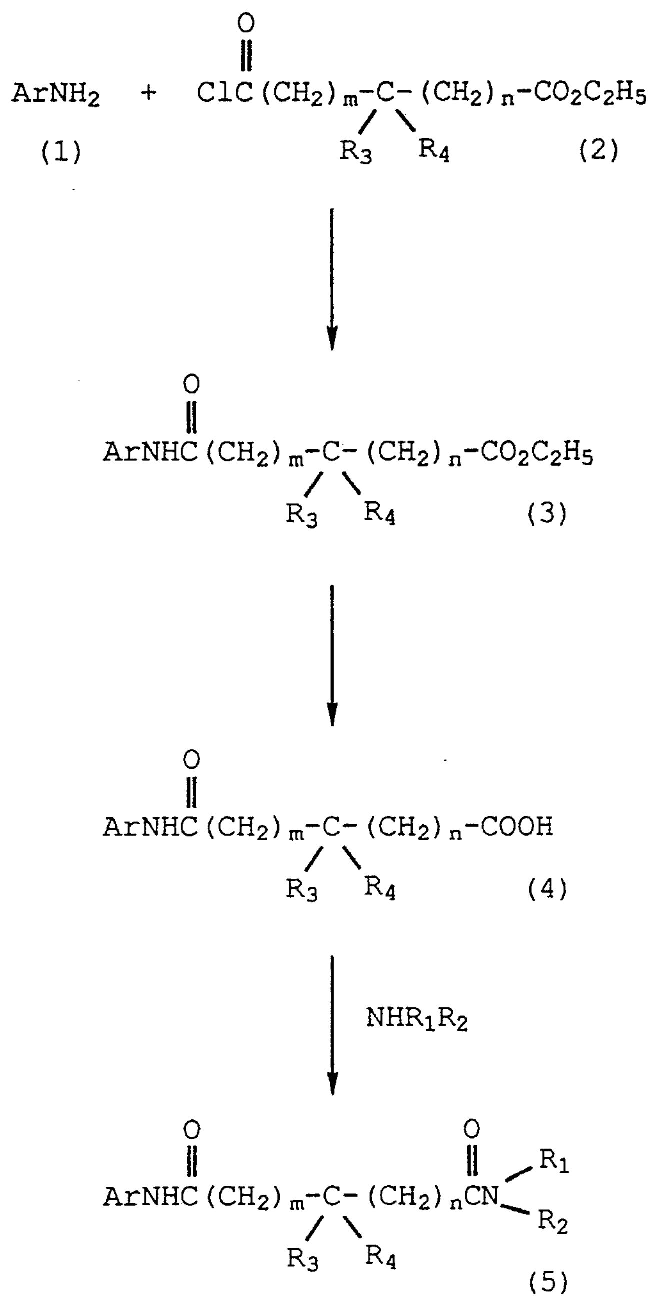


(1) (2)

(3)

(4)

(5)

CHART II

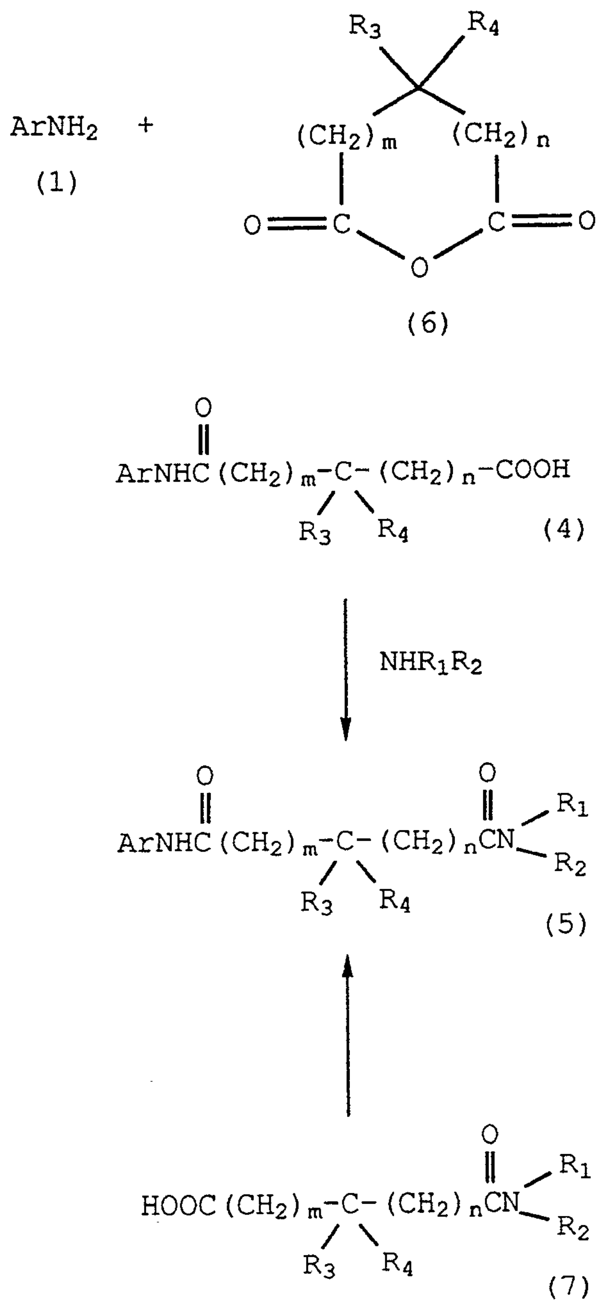


(1) (6) (4) (5) (7)

## Claims

1. A compound of the following general Formula I

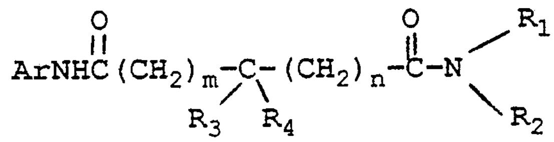

I

wherein Ar is

(a) phenyl$(CH_2)_x$- wherein X is zero to 2 and wherein the phenyl ring is unsubstituted or is substituted with from 1 to 3 substituents selected from

alkyl having from 1 to 6 carbon atoms and which is straight or branched,

alkoxy having from 1 to 6 carbon atoms and which is straight or branched,

phenoxy,

hydroxy,

fluorine,

chlorine,

bromine,

nitro,

trifluoromethyl,

-COOH,

-COOalkyl wherein alkyl has from 1 to 4 carbon atoms,

-$NR_5R_6$ wherein

$R_5$ and $R_6$ are independently hydrogen or alkyl of from 1 to 4 carbon atoms;

(b) 1- or 2-naphthyl which is unsubstituted or substituted with

alkyl having from 1 to 6 carbon atoms and which is straight or branched;

alkoxy having from 1 to 6 carbon atoms and which is straight or branched,

hydroxy,

fluorine,

chlorine,

bromine,

nitro,

trifluoromethyl,

-COOH,

-COOalkyl wherein alkyl has from 1 to 4 carbon atoms,

-$NR_5R_6$ wherein $R_5$ and $R_6$ have the meanings defined above;

wherein each of m and n is independently zero, one, or two;

wherein each of $R_3$ and $R_4$ is independently (a) hydrogen;

(b) a straight or branched alkyl group having from 1 to 10 carbon atoms;

(c) a straight chain alkyl group having from 1 to 10 carbon atoms wherein the terminal carbon atom is substituted with hydroxy or $NR_5R_6$ wherein $R_5$ and $R_6$ have the meanings defined above;

(d) $R_3$ is hydrogen and $R_4$ is the group -$NHR_{10}$ wherein $R_{10}$ is -C(=O)alkyl $C_{1-6}$ wherein the alkyl moiety is straight or branched; -(C=O)O-t-$C_4H_9$;

-C(=O)$OCH_2C_6H_5$;

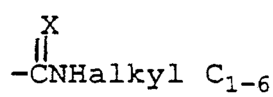

wherein X is oxygen or sulfur and the alkyl moiety is straight or branched;

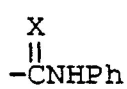

wherein X is oxygen or sulfur and Ph is phenyl or substituted phenyl wherein the substituents vary in number from 1 to 3 and are selected from chlorine, fluorine, bromine, iodine, trifluoromethyl, lower alkyl having from 1 to 4 carbon atoms and being straight or branched, lower alkoxy having from 1 to 4 carbon atoms and being straight or branched or $NR_5R_6$ wherein $R_5$ and $R_6$ have the meanings defined

hereinabove; or

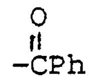

wherein Ph is phenyl or phenyl substituted with from 1 to 3 substituents selected from chlorine, fluorine, bromine, iodine, trifluoromethyl, lower alkyl having frmo 1 to 4 carbon atoms and being straight or branched, lower alkoxy having from 1 to 4 carbon atoms and being straight or branched, or $NR_5R_6$ wherein $R_5$ and $R_6$ have the meanings defined hereinabove; or

(e) $R_3$ is hydrogen and $R_4$ is $NR_{12}R_{13}$ wherein each of $R_{12}$ and $R_{13}$ is selected from hydrogen, straight or branched alkyl having from 1 to 6 carbon atoms, phenyl or benzyl or phenyl or benzyl substituted on any positions 2 through 6 of the aromatic ring with from 1 to 3 substituents selected from fluorine, chlorine, bromine, iodine, trifluoromethyl, lower alkyl having from 1 to 4 carbon and being straight or branched, lower alkoxy having from 1 to 4 carbon atoms and being straight or branched, or $NR_5R_6$ wherein $R_5$ and $R_6$ have the meanings defined hereinabove;

wherein each of $R_1$ and $R_2$ is independently

(a) hydrogen,

(b) the group

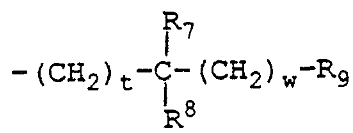

wherein t is zero to 4; w is zero to 4 with the proviso that the sum of t and w is not greater than 5; $R_7$ and $R_8$ are independently selected from hydrogen or alkyl having from 1 to 6 carbon atoms, or when $R_7$ is hydrogen, $R_8$ can be selected from the group defined for $R_9$; and $R_9$ is phenyl or phenyl substituted with from 1 to 3 substituents selected from straight or branched alkyl having from 1 to 6 carbon atoms, straight or branched alkoxy having from 1 to 6 carbon atoms, the group

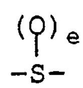

alkyl wherein e is zero, one or two, and alkyl is straight or branched and has from 1 to 6 carbon atoms, phenoxy, hydroxy, fluorine, chlorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above;

(c) the group

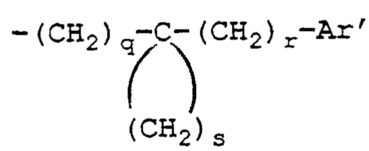

wherein q is zero to 3; r is zero to 2; s is 2 to 6; and Ar′ is

phenyl,

1- or 2-naphthyl,

phenyl or 1- or 2-naphthyl substituted with alkyl of from one to six carbon atoms,

alkoxy of from one to six carbon atoms,

hydroxy,

benzyloxy,

fluorine,

chlorine,

bromine,

nitro,

trifluoromethyl,
$-NH-COCH_3$,
$-CONH_2$,
-COOH,
-COOalkyl wherein alkyl has from one to four carbon atoms,
$-CH_2COOH$,
$-CH_2CONH_2$,
$-NR_5R_6$ wherein $R_5$ and $R_6$ have the meanings defined above;

(d) a straight or branched hydrocarbon chain having from 1 to 20 carbon atoms and which is saturated or contains from 1 to 3 double bonds;

(e) an alkyl group having from 1 to 6 carbon atoms wherein the terminal carbon is substituted with hydroxy, $-NR_5R_6$ where in $R_5$ and $R_6$ have the meanings defined above, or -COOalkyl wherein alkyl is straight or branched and has from 1 to 4 carbon atoms;

(f) $-(CH_2)_pQ$ wherein p is a number from zero to 3 and Q is a 5- or 6-membered monocyclic or fused bicyclic heterocycle containing at least 1 to 4 nitrogen, oxygen, or sulfur atoms in at least one ring member;

(g) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, $-NR_5R_6$ wherein $R_5$ and $R_6$ have the meanings defined above, hydroxy, nitro, chlorine, fluorine, bromine, or trifluoromethyl;

(h) $NR_1R_2$ taken together are phenothiazine or form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, ω-hydroxyalkyl having from 1 to 6 carbon atoms, phenyl, benzyl, or substituted phenyl or substituted benzyl wherein the substituents vary from 1 to 3 and can be on any position of 2 through 6 of the aromatic ring and are selected from straight or branched alkyl having from 1 to 4 carbon atoms, straight or branched alkoxy having from 1 to 4 carbon atoms, hydroxy, fluorine, chlorine, bromine, trifluoromethyl, or nitro; or

a pharmaceutically acceptable salt thereof.

2. A compound of Claim 1 wherein Ar is phenyl$(CH_2)_x$ wherein x is zero or two.

3. A compound of Claim 2 wherein the phenyl ring is unsubstituted.

4. A compound of Claim 2 wherein the phenyl ring is substituted.

5. A compound of Claim 4 wherein the phenyl ring is substituted on the 2,6-positions.

6. A compound of Claim 1 wherein each of $R_1$ and $R_2$ is independently hydrogen or the group:

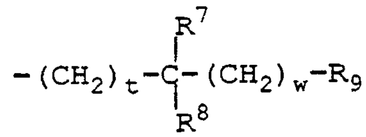

7. A compound of Claim 1 wherein each of $R_1$ and $R_2$ is independently hydrogen or the group:

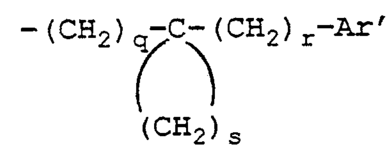

8. A compound of Claim 1 which is
N-[2,6-bis(1-methylethyl)phenyl]-N′-(2,2-diphenylethyl)propanediamide,
N-[2,6-bis(1-methylethyl)phenyl]-N′-(1-phenylcyclopentyl)methylpropanediamide,
N′-[2,6-bis(1-methylethyl)phenyl]-N′-(diphenylmethyl)propanediamide,
N′-[2,6-bis(1-methylethyl)phenyl]-N,N-bis-(phenylmethyl)propanediamide,
N′-[2,6-bis(1-methylethyl)phenyl]-N-(diphenylmethyl)-N-(1-methylethyl)propanediamide,
N-[2,6-bis(1-methylethyl)phenyl]-β-oxo-4-phenyl-1-piperazinepropanamide,
N-[2,6-bis(1-methylethyl)phenyl]-β-oxo-4-phenyl-1-piperidinepropanamide,
(S)-N-[2,6-bis(1-methylethyl)phenyl]-2-(hydroxymethyl)-β-oxo-1-pyrrolidinepropanamide,
N′-[2,6-bis(1-methylethyl)phenyl]-N-methyl-N-[2-(2-pyridyl)ethyl]propanediamide,

N′-[2,6-bis(1-methylethyl)phenyl]-N(1-methylethyl)-N-(phenylmethyl) propanediamide,
N,N-dipentyl-N′-[2,6-bis(1-methylethyl)phenyl]propanediamide,
N-[2,6-bis(1-methylethyl)phenyl]-N′-(phenylmethyl) propanediamide,
N,N-di-(2-hydroxyethyl)-N′-[2,6-bis(1-methylethyl)phenyl)propanediamide,
N′-[2,6-bis(1-methylethyl)phenyl]-N-[(4-fluorophenyl)methyl]-N-(1-methylethyl)propanediamide,
N-[2,6-bis(1-methylethyl)phenyl-N′-(2-dimethylaminoethyl)-N′-(phenylmethyl)propanediamide,
N-[2,6-bis(1-methylethyl)phenyl]-N′-(2-hydroxyethyl)-N′-(phenylmethyl)propanediamide,
N-[2,6-bis(1-methylethyl)phenyl]-N′-(ethyl)-N′-(2-diethylaminoethyl)propanediamide,
N-[2,6-bis(1-methylethyl)phenyl]-β-oxo-4-(2-hydroxyethyl)-1-piperazinepropanamide,
N-[2,6-bis(1-methylethyl)phenyl]-β-oxo-1-morpholinepropanamide,
N-[2,6-bis(1-methylethyl)phenyl]-β-oxo-1-thiomorpholinepropanamide,
N-[2,6-bis(1-methylethyl)phenyl]-N′-[2-cyanoethyl)-N′-(phenylmethyl)propanediamide,
N-[2,6-bis(1-methylethyl)phenyl]-N′-(diphenylmethyl)pentanamide,
N′-[2,6-bis(1-methylethyl)phenyl]-N-(1-methylethyl)-N[[4-(methylsulfinyl)phenyl]methyl]propanediamide;
N′-[2,6-bis(1-methylethyl)phenyl]-2,2-dimethyl-N-(1-methylethyl)-N-(phenylmethyl)propanediamide,
N′-[2,6-bis(1-methylethyl)phenyl]-N(1-methylethyl)-N-phenylpropanediamide,
N-[2,6-bis(1-methylethyl)phenyl]-N-(1-methylethyl)-N-[(3-pyridinyl)methyl]propanediamide,
N-[2,6-bis(1-methylethyl)phenyl]-β-oxo-4-(phenylmethyl)-1-piperazinepropanamide,
N′-[2,6-bis(1-methylethyl)phenyl]-N-(1-methylethyl)-N-[[4-(methylthio)phenyl]methyl] propanediamide,
N-[3-[[2,6-bis(1-methylethyl)phenyl]amino]-1,3-dioxopropyl]-N-phenylglycine, ethyl, ester;
N-[2,6-bis(1,1-dimethylethyl)phenyl]-4-(2-methoxyphenyl)-β-oxo-1-piperazinepropanamide,
N-[2,6-bis(1-methylethyl)phenyl]-1-(diphenylmethyl)1-piperazinepropanamide,
N-[2,6-bis(1-methylethyl)phenyl]-β-[[(4-methoxyphenyl)methyl] (2-pyridinyl)amino]-β-oxopropanamide,
N-[2,6-bis(1-methylethyl)phenyl]-β-oxo-10H-phenothiazine-10-propanamide,
N′-[2,6-bis(1-methylethyl)phenyl]-N-[(4-methoxyphenyl)methyl]-N-(2-pyrimidinyl)-propanediamide,
N-[2,6-bis(1-methylethyl)phenyl]-N′-heptylpropanediamide,
N,N′-bis[2,6-bis(1-methylethyl)phenyl]propanediamide,
N′-[2,6-bis(1-methylethyl)phenyl]-N-[[4-(dimethylamino)phenyl]methyl]-N-(1-methylethyl)propanediamide,
N-[2,6-bis(1-methylethyl)phenyl]-N′-(1,1,3,3-tetramethylbutyl) propanediamide,
N′-[2,6-bis(1-methylethyl)phenyl]-N-methyl-N-octylpropanediamide,
N-[2,6-bis(1-methylethyl)phenyl]-N′-(1,1-dimethyl-2-phenylethyl) propanediamide,
N-[2,6-bis(dimethylamino)phenyl]-2,6-bis(1-methylethyl)phenyl]propanediamide, and N′-[2,6-bis(1-methylethyl)phenyl]-N-methyl-N-octadecylpropanediamide

9. A process for preparing a compound of formula I

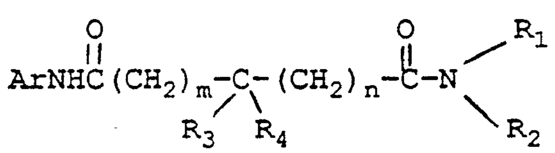

I

wherein Ar is
(a) phenyl$(CH_2)_x$- wherein X is zero to 2 and wherein the phenyl ring is unsubstituted or is substituted with from 1 to 3 substituents selected from
alkyl having from 1 to 6 carbon atoms and which is straight or branched,
alkoxy having from 1 to 6 carbon atoms and which is straight or branched,
phenoxy,
hydroxy,
fluorine,
chlorine,
bromine,
nitro,
trifluoromethyl,
-COOH,
-COOalkyl wherein alkyl has from 1 to 4 carbon atoms,
$-NR_5R_6$ wherein
$R_5$ and $R_6$ are independently hydrogen or alkyl of from 1 to 4 carbon atoms;

(b) 1- or 2-naphthyl which is unsubstituted or substituted with
alkyl having from 1 to 6 carbon atoms and which is straight or branched;
alkoxy having from 1 to 6 carbon atoms and which is straight or branched,
hydroxy,
fluorine,
chlorine,
bromine,
nitro,
trifluoromethyl,
-COOH,
-COOalkyl wherein alkyl has from 1 to 4 carbon atoms,
$-NR_5R_6$ wherein $R_5$ and $R_6$ have the meanings defined above;
wherein each of m and n is independently zero, one, or two;
wherein each of $R_3$ and $R_4$ is independently (a) hydrogen;
(b) a straight or branched alkyl group having from 1 to 10 carbon atoms;
(c) a straight chain alkyl group having from 1 to 10 carbon atoms wherein the terminal carbon atom is substituted with hydroxy or $NR_5R_6$ wherein $R_5$ and $R_6$ have the meanings defined above;
(d) $R_3$ is hydrogen and $R_4$ is the group $-NHR_{10}$ wherein $R_{10}$ is -C(=O)alkyl $C_{1-6}$ wherein the alkyl moiety is straight or branched; $-(C=O)O-t-C_4H_9$; $-C(=O)OCH_2C_6H_5$;

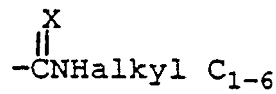

wherein X is oxygen or sulfur and the alkyl moiety is straight or branched;

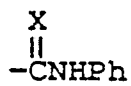

wherein X is oxygen or sulfur and Ph is phenyl or substituted phenyl wherein the substituents vary in number from 1 to 3 and are selected from chlorine, fluorine, bromine, iodine, trifluoromethyl, lower alkyl having from 1 to 4 carbon atoms and being straight or branched, lower alkoxy having from 1 to 4 carbon atoms and being straight or branched or $NR_5R_6$ wherein $R_5$ and $R_6$ have the meanings defined hereinabove; or

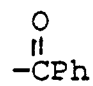

wherein Ph is phenyl or phenyl substituted with from 1 to 3 substituents selected from chlorine, fluorine, bromine, iodine, trifluoromethyl, lower alkyl having frmo 1 to 4 carbon atoms and being straight or branched, lower alkoxy having from 1 to 4 carbon atoms and being straight or branched, or $NR_5R_6$ wherein $R_5$ and $R_6$ have the meanings defined hereinabove; or
(e) $R_3$ is hydrogen and $R_4$ is $NR_{12}R_{13}$ wherein each of $R_{12}$ and $R_{13}$ is selected from hydrogen, straight or branched alkyl having from 1 to 6 carbon atoms, phenyl or benzyl or phenyl or benzyl substituted on any positions 2 through 6 of the aromatic ring with from 1 to 3 substituents selected from fluorine, chlorine, bromine, iodine, trifluoromethyl, lower alkyl having from 1 to 4 carbon and being straight or branched, lower alkoxy having from 1 to 4 carbon atoms and being straight or branched, or $NR_5R_6$ wherein $R_5$ and $R_6$ have the meanings defined hereinabove; wherein each of $R_1$ and $R_2$ is independently
(a) hydrogen,
(b) the group

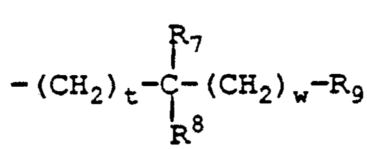

wherein t is zero to 4; w is zero to 4 with the proviso that the sum of t and w is not greater than 5; $R_7$ and $R_8$ are independently selected from hydrogen or alkyl having from 1 to 6 carbon atoms, or when $R_7$ is hydrogen, $R_8$ can be selected from the group defined for $R_9$; and $R_9$ is phenyl or phenyl substituted with from 1 to 3 substituents selected from straight or branched alkyl having from 1 to 6 carbon atoms, straight or branched alkoxy having from 1 to 6 carbon atoms, the group

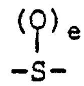

alkyl wherein e is zero, ore or two, and alkyl is straight or branched and has from 1 to 6 carbon atoms, phenoxy, hydroxy, fluorine, chlorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above;

(c) the group

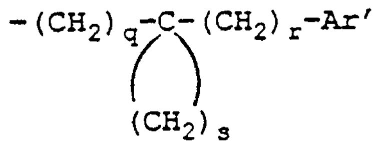

wherein q is zero to 3; r is zero to 2; s is 2 to 6; and Ar′ is
phenyl,
1- or 2-naphthyl,
phenyl or 1- or 2-naphthyl substituted with alkyl of from one to six carbon atoms,
alkoxy of from one to six carbon atoms,
hydroxy,
benzyloxy,
fluorine,
chlorine,
bromine,
nitro,
trifluoromethyl,
$-NH-COCH_3$,
$-CONH_2$,
-COOH,
-COOalkyl wherein alkyl has from one to four carbon atoms,
$-CH_2COOH$,
$-CH_2CONH_2$,
$-NR_5R_6$ wherein $R_5$ and $R_6$ have the meanings defined above;

(d) a straight or branched hydrocarbon chain having from 1 to 20 carbon atoms and which is saturated or contains from 1 to 3 double bonds;

(e) an alkyl group having from 1 to 6 carbon atoms wherein the terminal carbon is substituted with hydroxy, $-NR_5R_6$ wherein $R_5$ and $R_6$ have the meanings defined above, or -COOalkyl wherein alkyl is straight or branched and has from 1 to 4 carbon atoms;

(f) $-(CH_2)_pQ$ wherein p is a number from zero to 3 and Q is a 5- or 6-membered monocyclic or fused bicyclic heterocycle containing at least 1 to 4 nitrogen, oxygen, or sulfur atoms in at least one ring member;

(g) phenyl or phenyl substituted with from 1 to 3 substituents selected from straight or branched alkyl having from 1 to 6 carbon atoms, alkoxy which is straight or branched and has from 1 to 6 carbon atoms, alkylthio which is straight or branched and has from 1 to 6 carbon atoms, $-NR_5R_6$ wherein $R_5$ and $R_6$ have the meanings defined above, hydroxy, nitro, chlorine, fluorine, bromine, or trifluoromethyl;

(h) $NR_1R_2$ taken together are phenothiazine or form a monocyclic heterocyclic group selected from pyrrolidino, piperidino, morpholino, or piperazino, each of which is unsubstituted or is substituted with one substituent selected from benzhydryl, straight or branched alkyl having from 1 to 6 carbon atoms, ω-hydroxyalkyl having from 1 to 6 carbon atoms, phenyl, benzyl, or substituted phenyl or substituted benzyl wherein the substituents vary from 1 to 3 and can be on any position of 2 through 6 of the aromatic ring

and are selected from straight or branched alkyl having from 1 to 4 carbon atoms, straight or branched alkoxy having from 1 to 4 carbon atoms, hydroxy, fluorine, chlorine, bromine, trifluoromethyl, or nitro;
or of a pharmaceutically acceptable salt thereof, which comprises reacting an arylamine of the formula $ArNH_2$
wherein Ar has the above meaning with an acid chloride of the formula

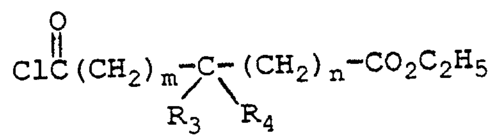

wherein m, n, $R_3$ and $R_4$ have the above meaning in an aprotic nonpolar solvent in the presence of a tertiary amine to give an ester amide of the formula

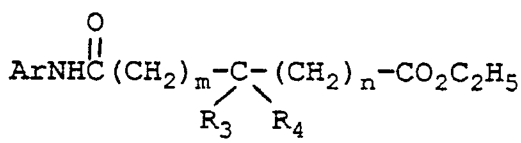

(b)

wherein n, m, Ar, $R_3$ and $R_4$ have the above meaning which is treated with a mild aqueous base to give an acid amide of the formula

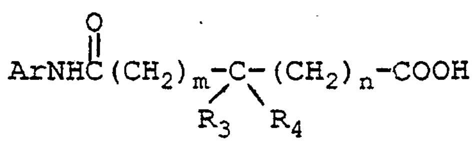

(c)

wherein n, m, Ar and $R_3$ and $R_4$ have the above meaning which is converted to the bis amides of formula (d) by reaction with an amine of the formula $HNR_1R_2$

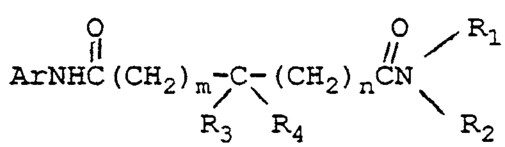

(d)

wherein Ar, $R_1$, $R_2$, $R_3$, $R_4$, m, and n have the above meaning except that $R_4$ is other than $NHR_{10}$ or $NR_{12}R_{13}$,
or alternatively reacting an arylamine of the formula $ArNH_2$ wherein Ar has the above meaning with an acid anhydride of the formula

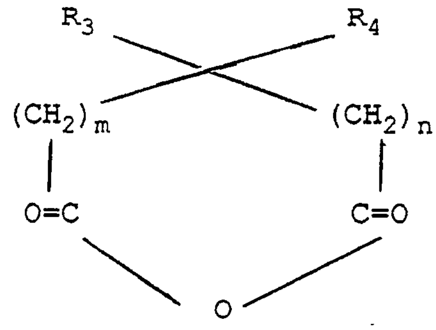

wherein $R_3$, $R_4$, m and n have the above meaning in an aprotic nonpolar solvent to give an amide acid of the formula (c) above which is converted to the bis-amide of formula (d) as defined above,
and when a compound wherein $R_4$ is $NHR_{10}$ or $NR_{12}R_{13}$, wherein $R_{10}$, $R_{12}$ and $R_{13}$ have the above meaning, is desired, reacting a compound of the formula

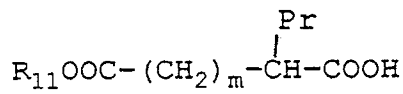

wherein m has the above meaning, $R_{11}$ is lower alkyl and Pr is tert-butoxycarbonyl or benzyloxycarbonyl with a carbodiimide followed by the addition of an amine of the formula $HNR_1R_2$
wherein $R_1$ and $R_2$ are as defined above to give an ester amide of the formula

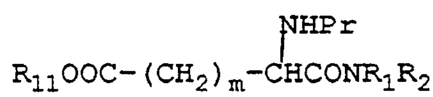

wherein $R_1$, $R_2$, $R_{11}$, Pr and m have the above meaning which is hydrolyzed to the corresponding amide acid of the formula

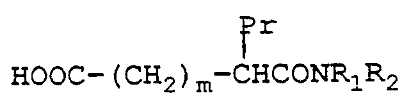

wherein m, $R_1$, $R_2$ and Pr have the above meaning, which is treated with a carbodiimide and reacted with an amine of the formula $ArNH_2$
wherein Ar has the above meaning to give compounds of the formula

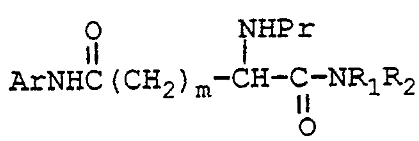

wherein $R_1$, $R_2$, Pr, m and Ar are as defined above which can be deprotected and acylated or alkylated, to give compounds of Claim 1 wherein $R_4$ is $NHR_{10}$ or $NR_{12}R_{13}$, further, when $R_3$ and $R_4$ are hydrogen a compound of formula I if desired is treated with a mild base and then alkylated with a compound of the formulae
Hal-$R_3$ and/or Hal-$R_4$
wherein Hal is a halogen atom and $R_3$ and $R_4$ as defined above,
and if desired a pharmaceutically acceptable salt is formed with an organic or anorganic acid.

10. A process of Claim 9 for preparing a compound as as defined in anyone of Claims 2 to 8.

11. A pharmaceutical composition containing a compound as defined in any of Claims 1 to 8 or as prepared

by the process of Claims 9 or 10 together with a pharmaceutically acceptable carrier.

12. Use of a compound as defined in any of Claims 1 to 8 or as prepared by the process of Claims 9 or 10 for the preparation of a pharmaceutical composition useful for treating hypercholesterolemia and atherosclerosis.

# F I G. 1

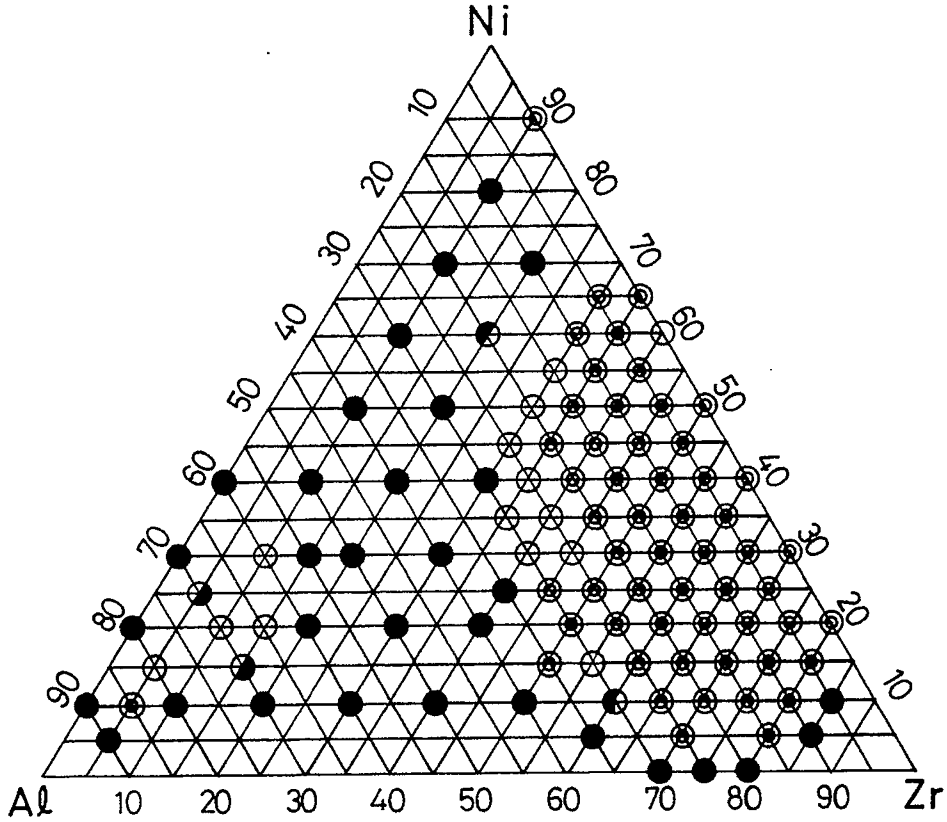
Ni
Aℓ
Zr
10 20 30 40 50 60 70 80 90

# F I G. 2

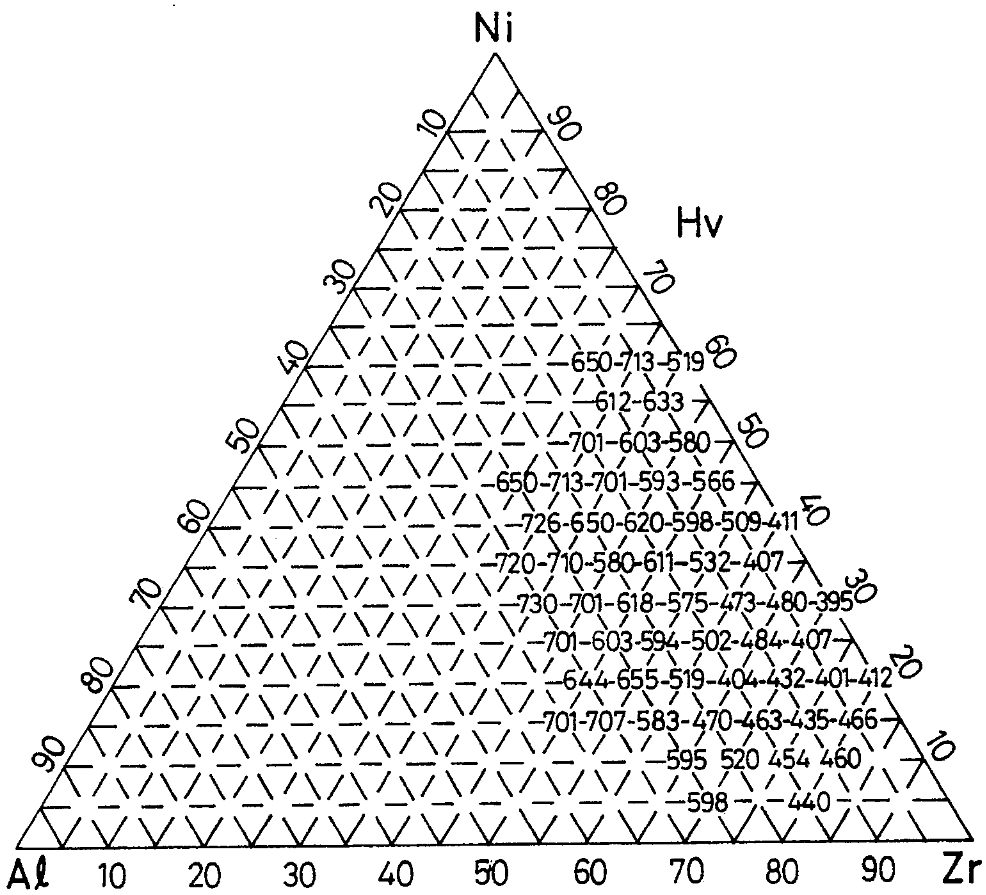
Ni
Hv
Aℓ
Zr
10 20 30 40 50 60 70 80 90
650-713-519
612-633
701-603-580
650-713-701-593-566
726-650-620-598-509-411
720-710-580-611-532-407
730-701-618-575-473-480-395
701-603-594-502-484-407
644-655-519-404-432-401-412
701-707-583-470-463-435-466
595 520 454 460
598 440

FIG. 3

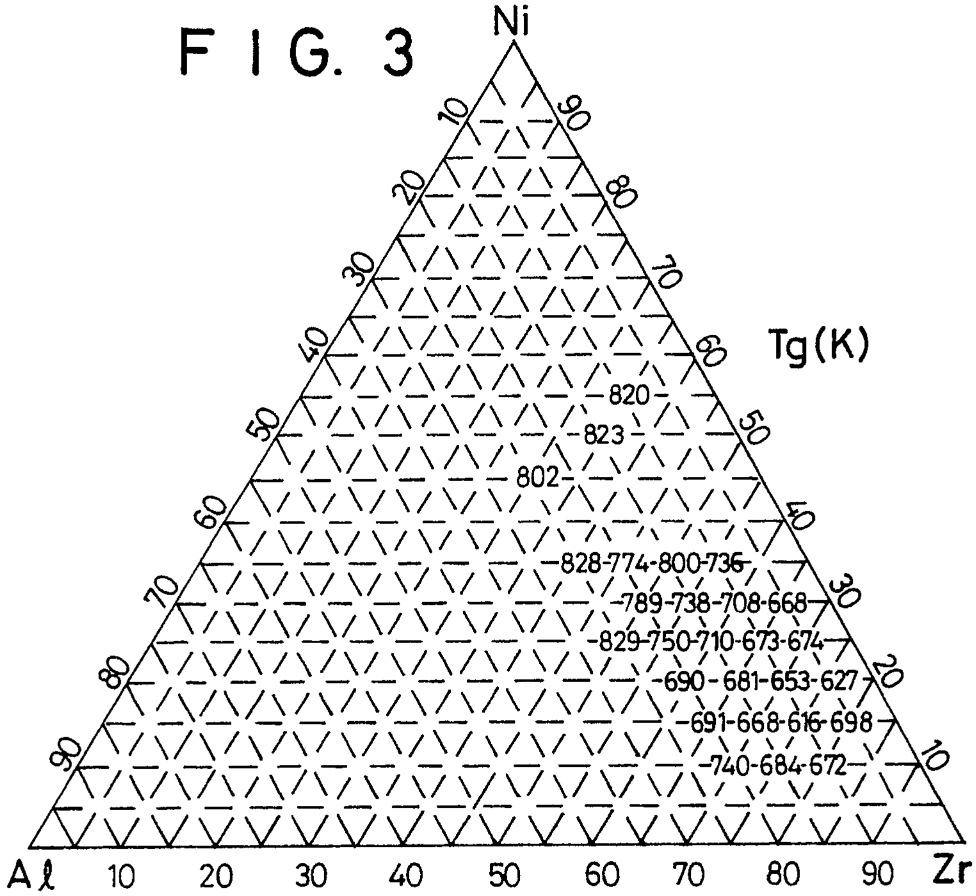
Ni
Tg(K)
820
823
802
828-774-800-736
789-738-708-668
829-750-710-673-674
690-681-653-627
691-668-616-698
740-684-672
Aℓ 10 20 30 40 50 60 70 80 90 Zr

FIG. 4

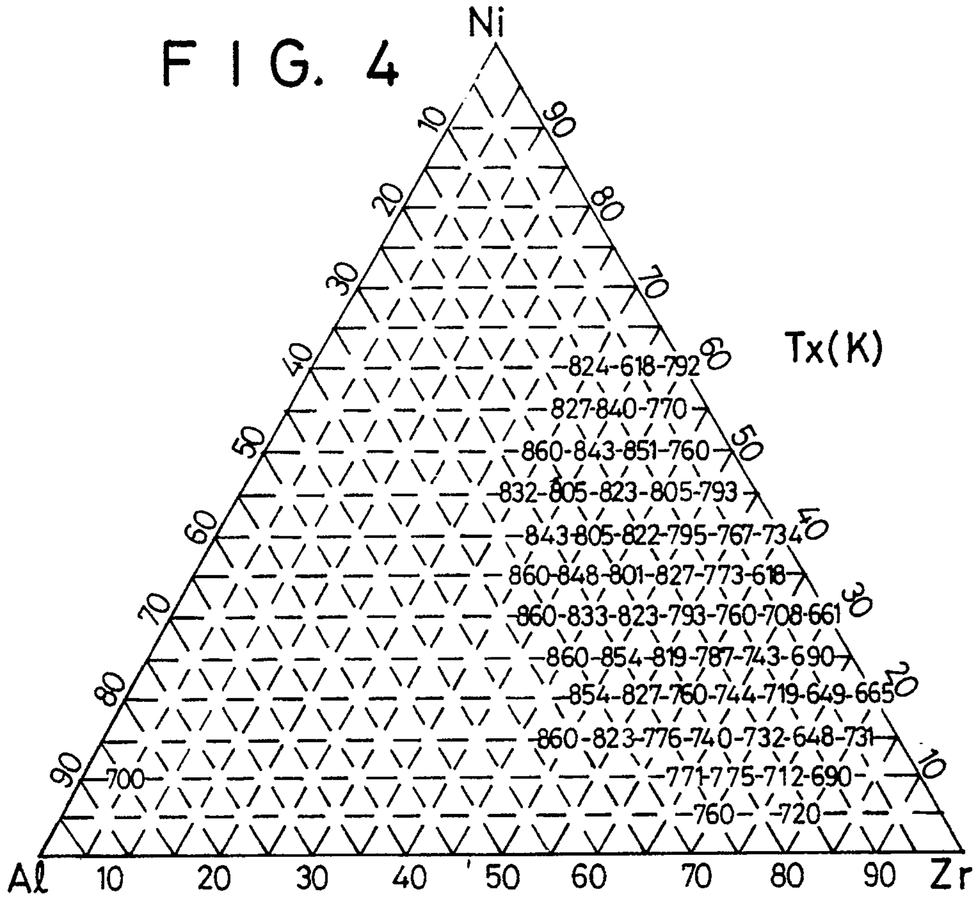
Ni
Tx(K)
824-618-792
827-840-770
860-843-851-760
832-805-823-805-793
843-805-822-795-767-734
860-848-801-827-773-618
860-833-823-793-760-708-661
860-854-819-787-743-690
854-827-760-744-719-649-665
860-823-776-740-732-648-731
700
771-775-712-690
760 720
Aℓ 10 20 30 40 50 60 70 80 90 Zr

# F I G. 5

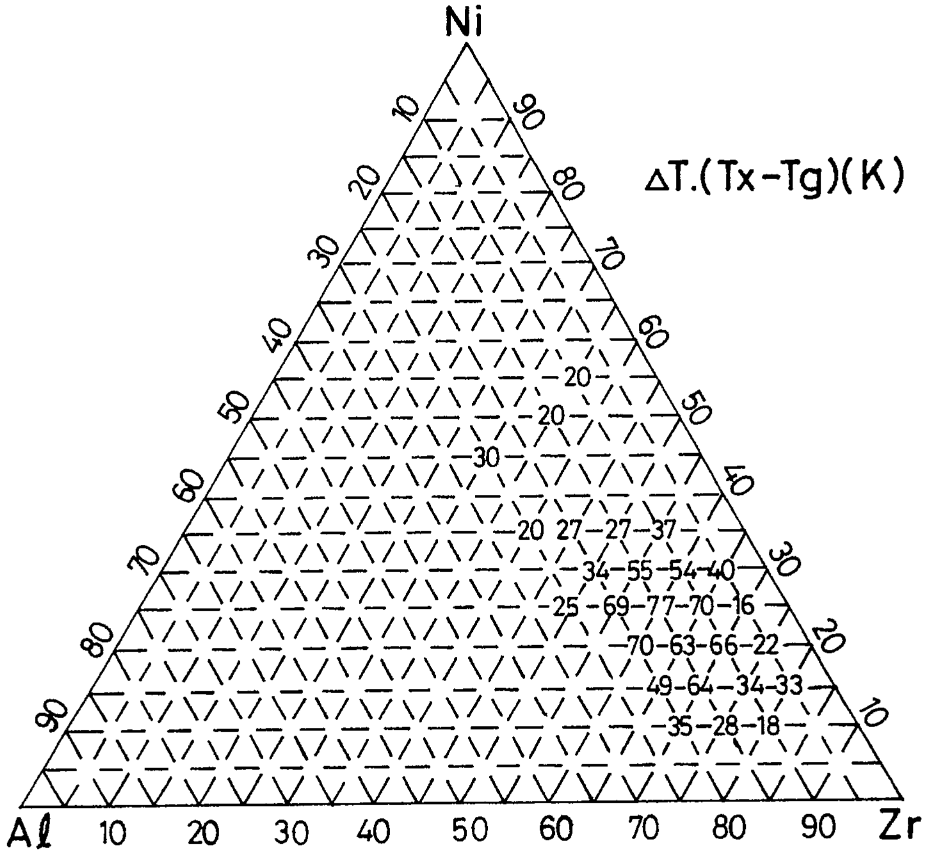
Ni
ΔT.(Tx−Tg)(K)
20
20
30
20 27−27−37
34−55−54−40
25−69−77−70−16
70−63−66−22
49−64−34−33
35−28−18
Al
Zr

# F I G. 6

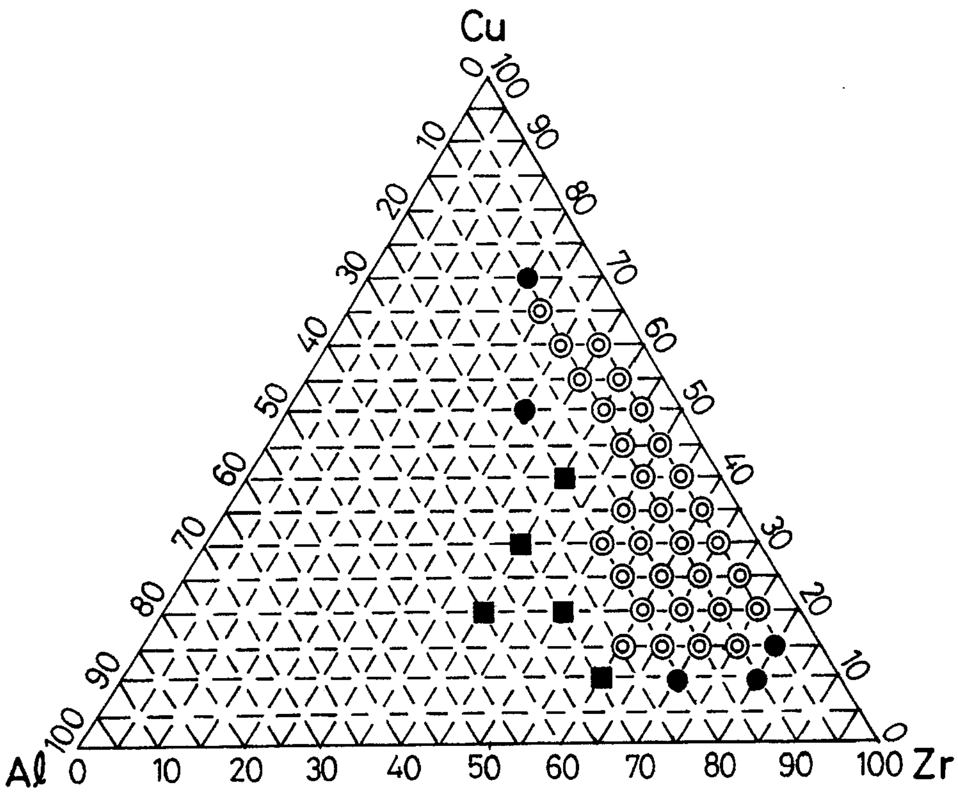
Cu
Al
Zr

FIG. 7

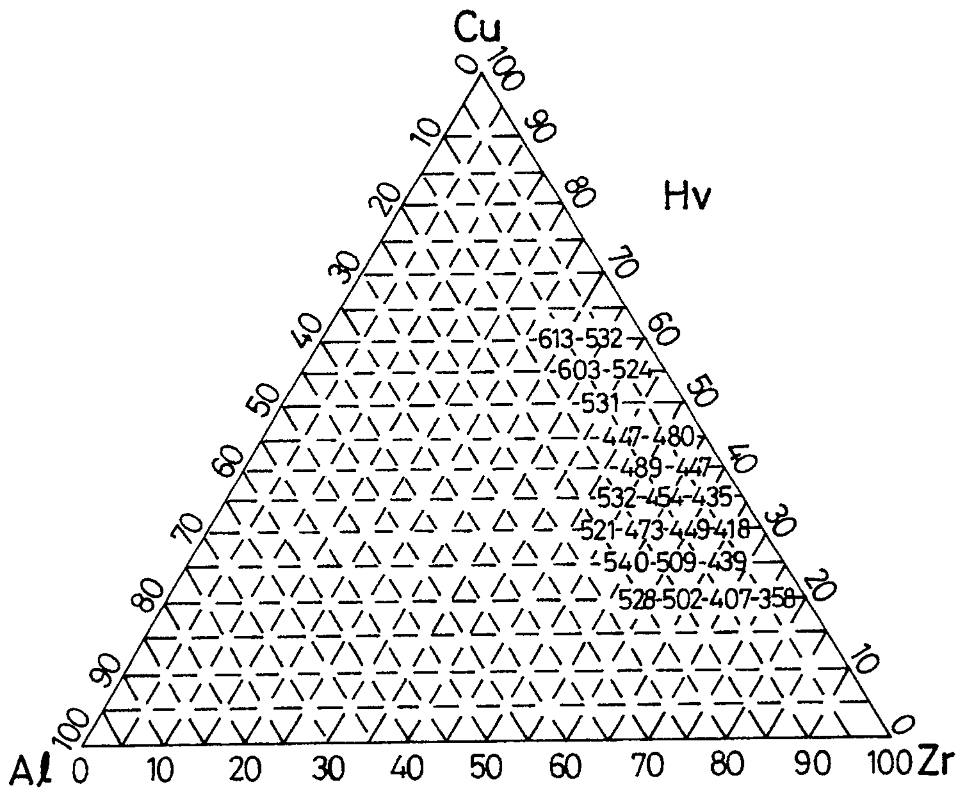
Cu
Hv
Al
Zr
613-532
603-524
531
447-480
489-447
532-454-435
521-473-449-418
540-509-439
528-502-407-358

FIG. 8

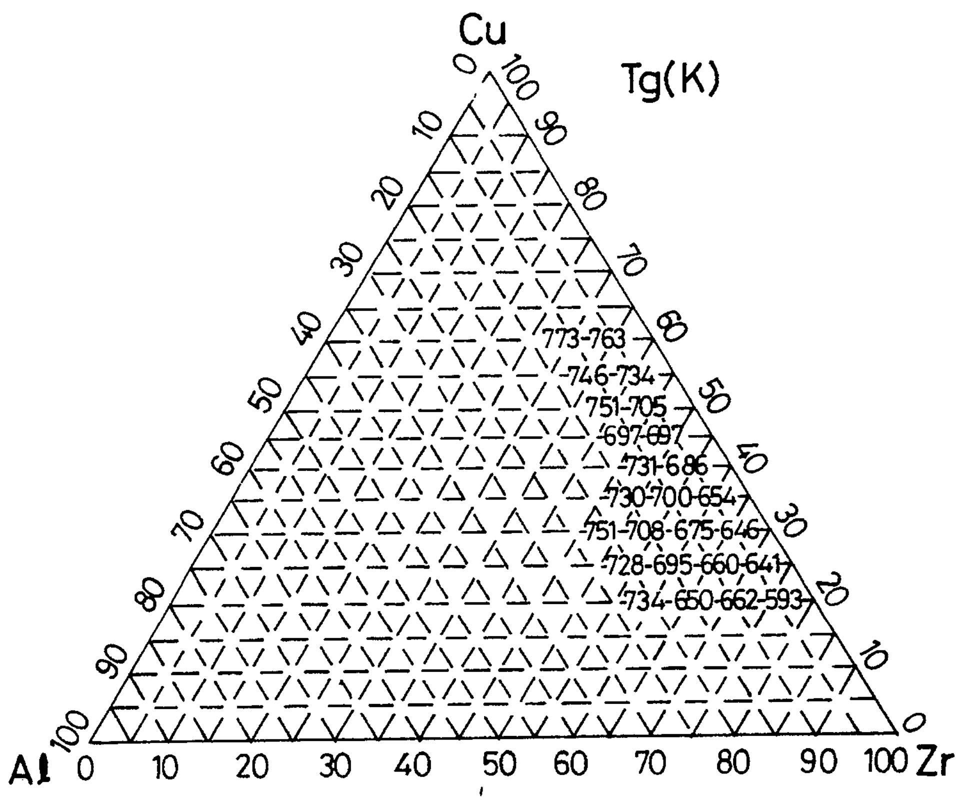
Cu
Tg(K)
Al
Zr
773-763
746-734
751-705
697-697
731-686
730-700-654
751-708-675-646
728-695-660-641
734-650-662-593

# F I G. 9

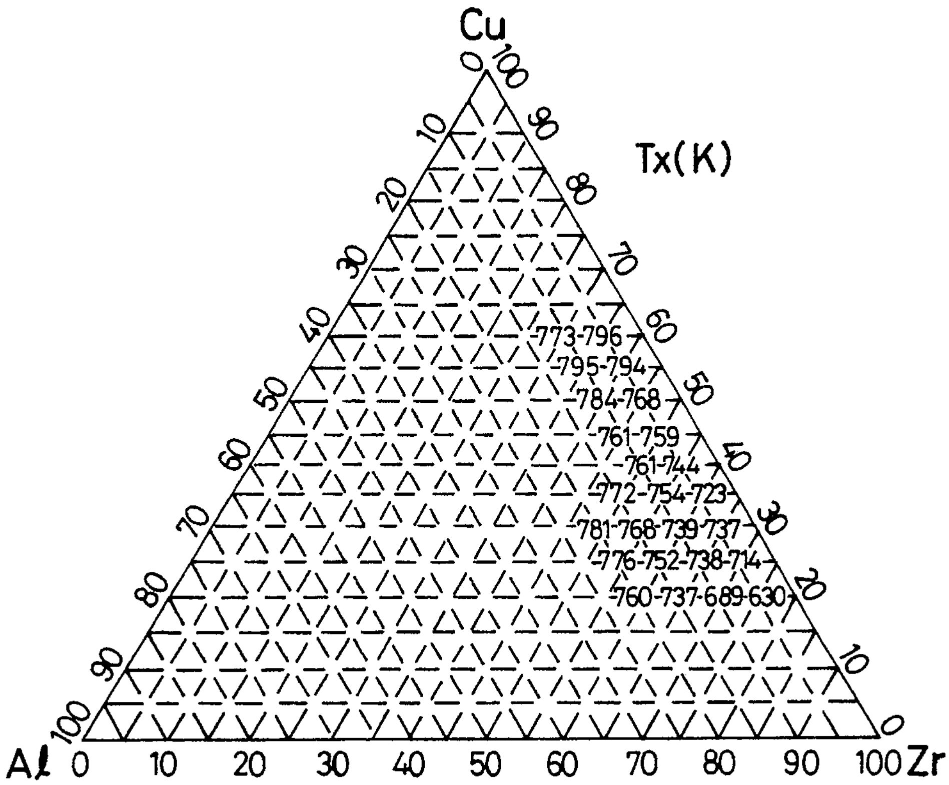
Cu
Tx(K)
773-796
795-794
784-768
761-759
761-744
772-754-723
781-768-739-737
776-752-738-714
760-737-689-630
Al
Zr
0
10
20
30
40
50
60
70
80
90
100

# F I G. 10

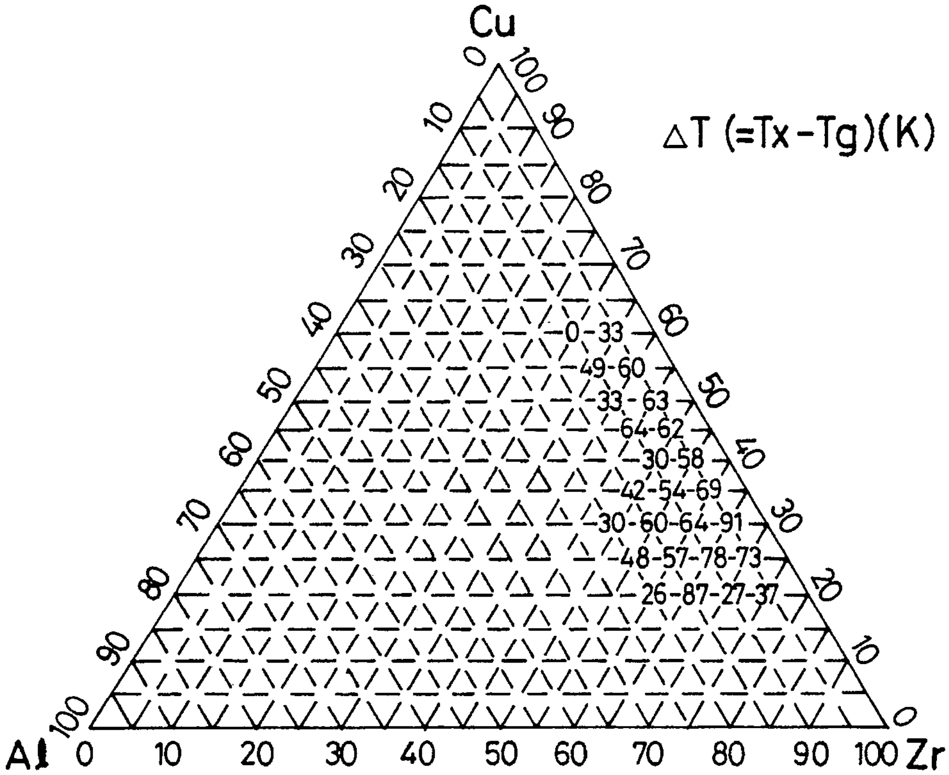
Cu
ΔT (=Tx-Tg)(K)
0-33
49-60
33-63
64-62
30-58
42-54-69
30-60-64-91
48-57-78-73
26-87-27-37
Al
Zr
0
10
20
30
40
50
60
70
80
90
100

# FIG. 11

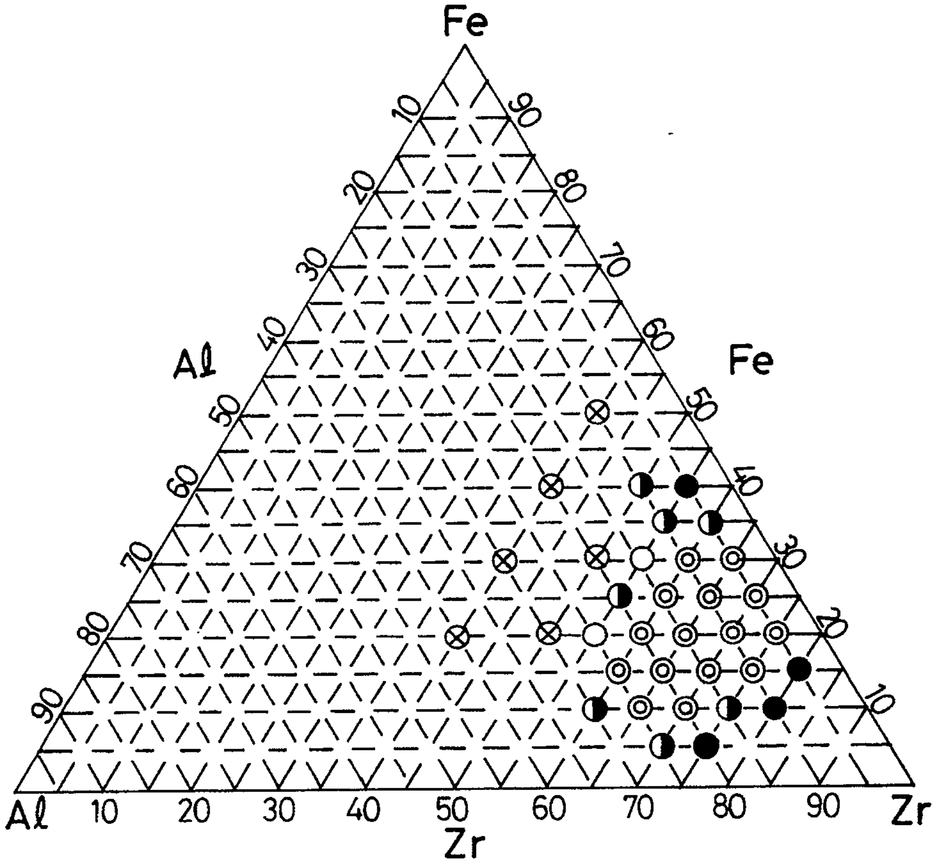
Fe
Al
Fe
10
20
30
40
50
60
70
80
90
Al
Zr
Zr

# FIG. 12

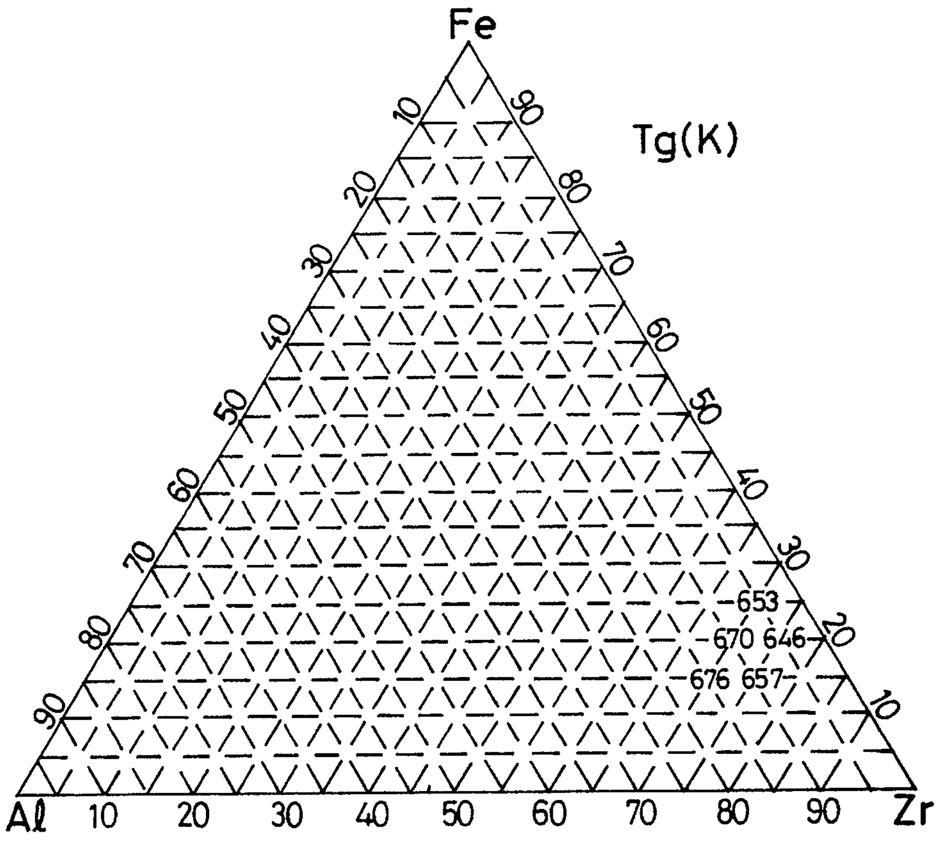
Fe
Tg(K)
653
670 646
676 657
10
20
30
40
50
60
70
80
90
Al
Zr

# F I G. 13

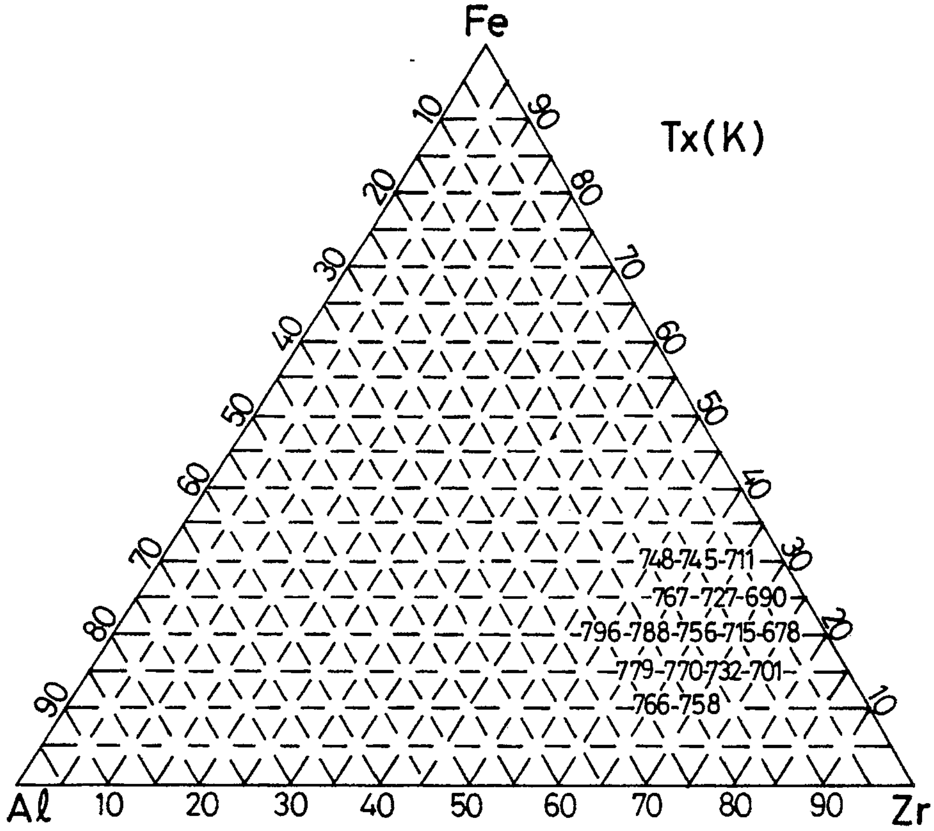
Fe
Tx(K)
748-745-711
767-727-690
796-788-756-715-678
779-770-732-701
766-758
Al
Zr
10
20
30
40
50
60
70
80
90

# F I G. 14

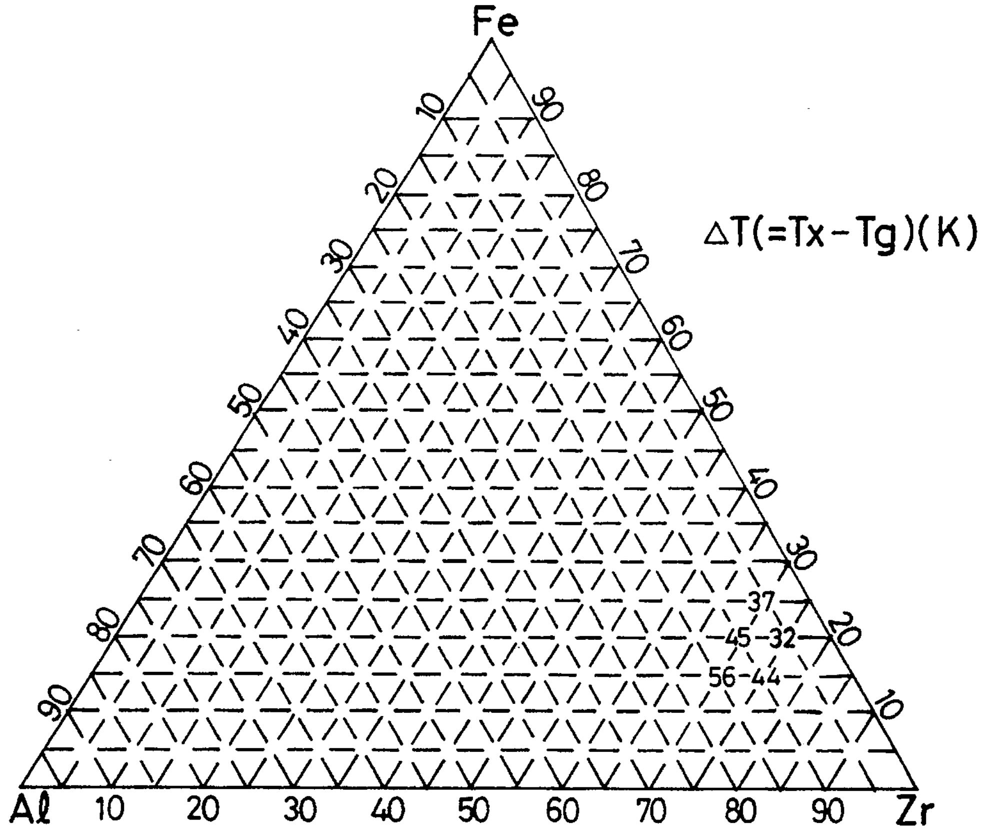
Fe
ΔT(=Tx−Tg)(K)
37
45-32
56-44
Al
Zr
10
20
30
40
50
60
70
80
90

F I G. 15

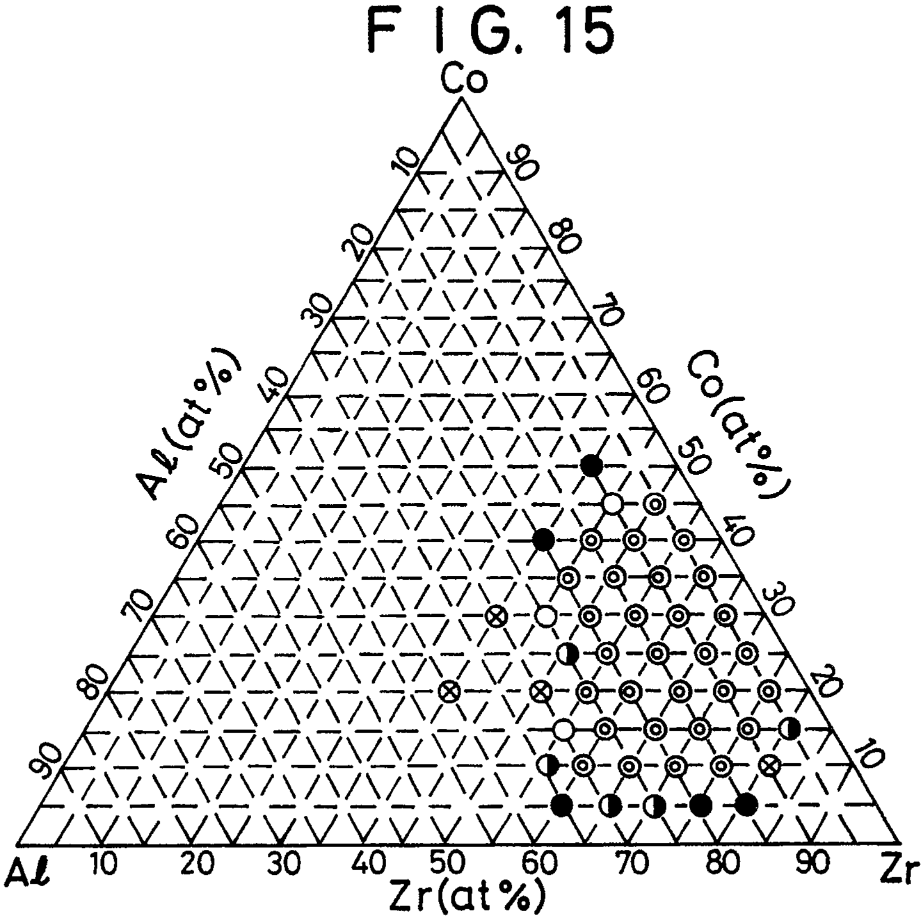
Co
Aℓ(at%)
Co(at%)
Aℓ
Zr
Zr(at%)
10
20
30
40
50
60
70
80
90

F I G. 16

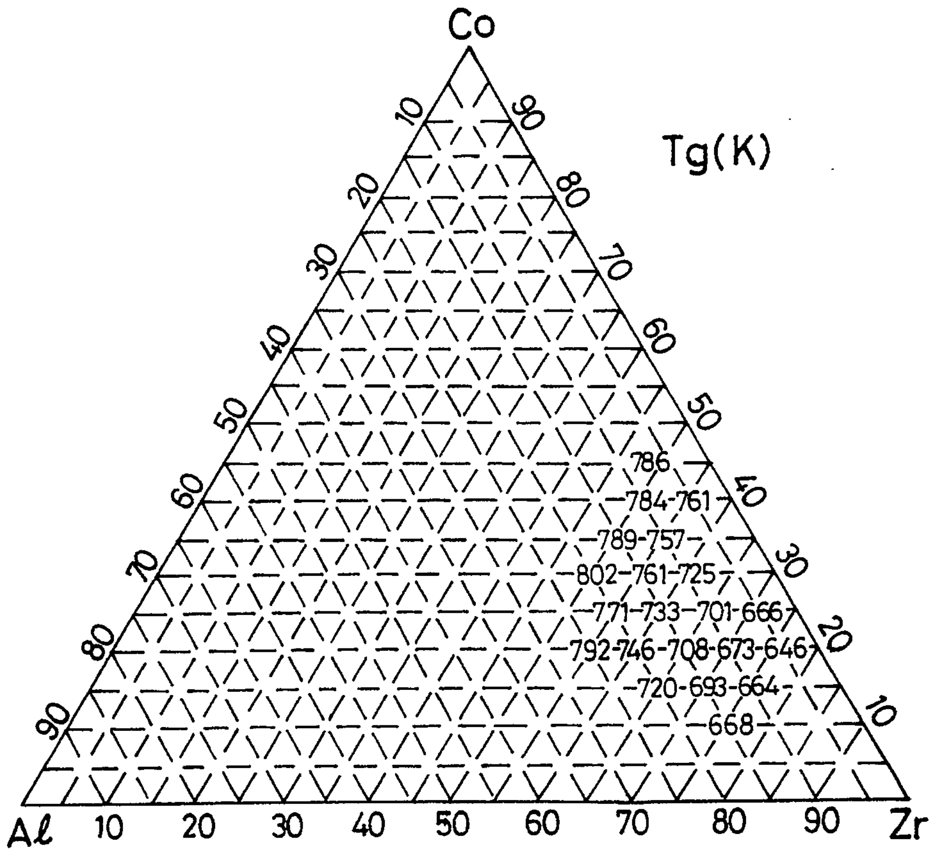
Co
Tg(K)
786
784-761
789-757
802-761-725
771-733-701-666
792-746-708-673-646
720-693-664
668
Aℓ
Zr
10
20
30
40
50
60
70
80
90

# F I G. 17

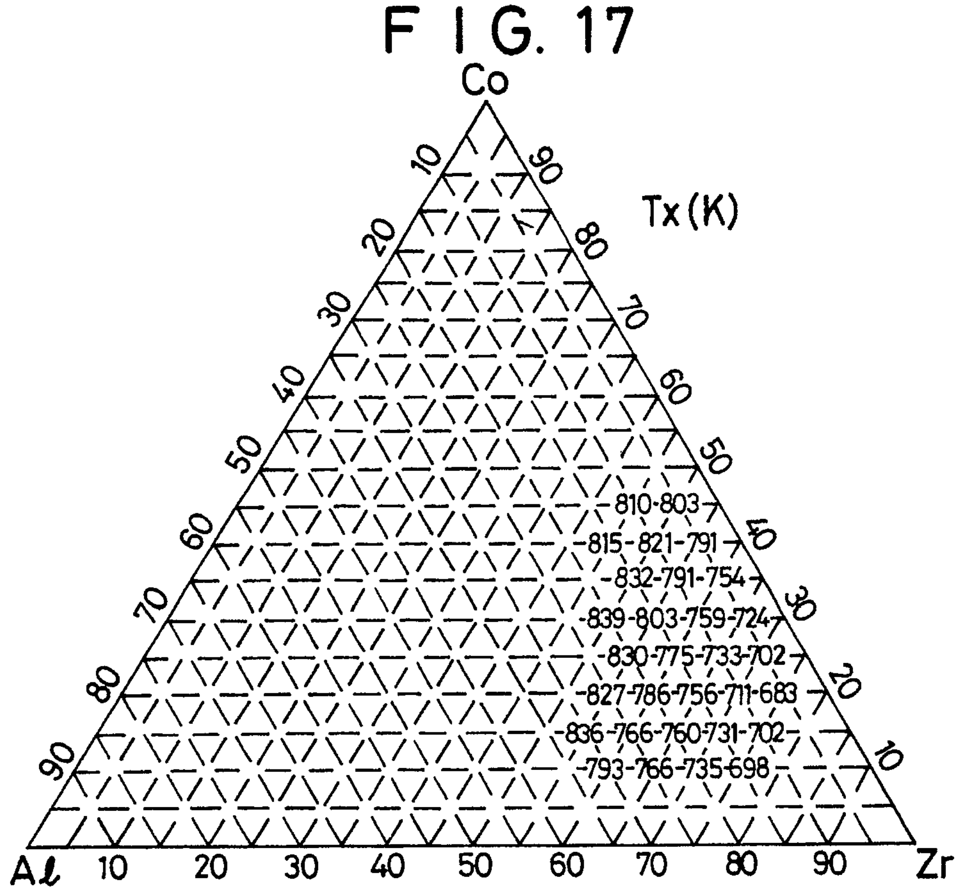
Co
Tx (K)
10
20
30
40
50
60
70
80
90
810-803
815-821-791
832-791-754
839-803-759-724
830-775-733-702
827-786-756-711-683
836-766-760-731-702
793-766-735-698
Al
10 20 30 40 50 60 70 80 90
Zr

# F I G. 18

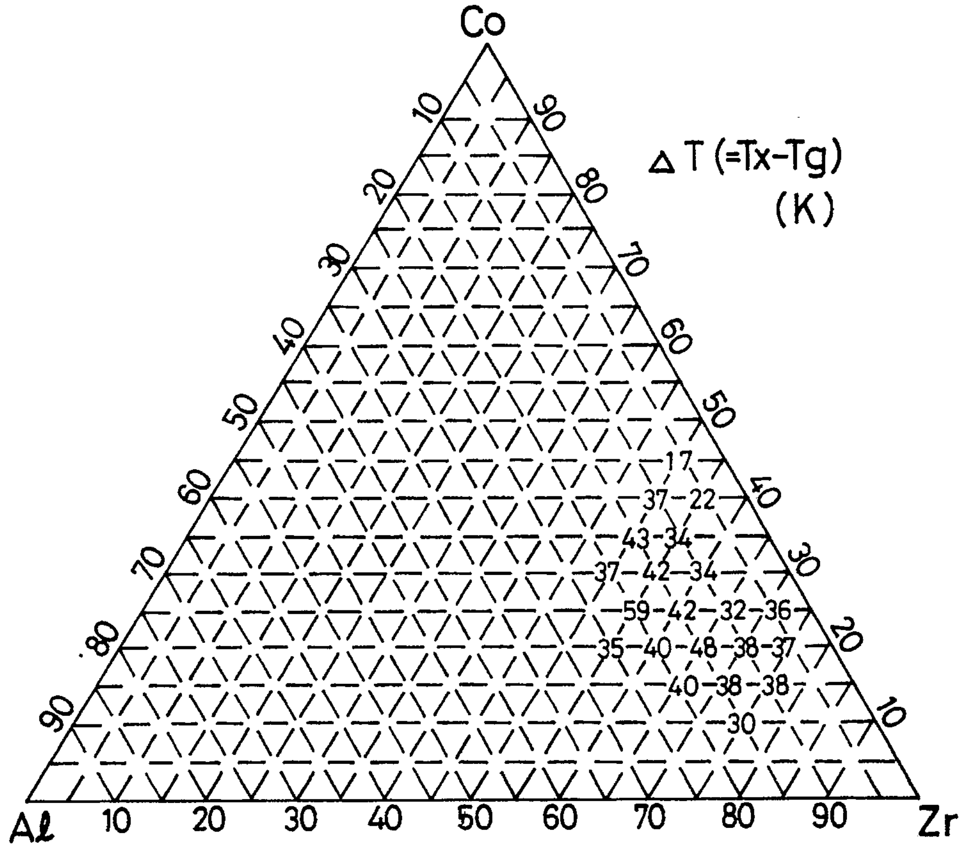
Co
ΔT (=Tx-Tg)
(K)
10
20
30
40
50
60
70
80
90
17
37-22
43-34
37-42-34
59-42-32-36
35-40-48-38-37
40-38-38
30
Al
10 20 30 40 50 60 70 80 90
Zr

FIG. 19

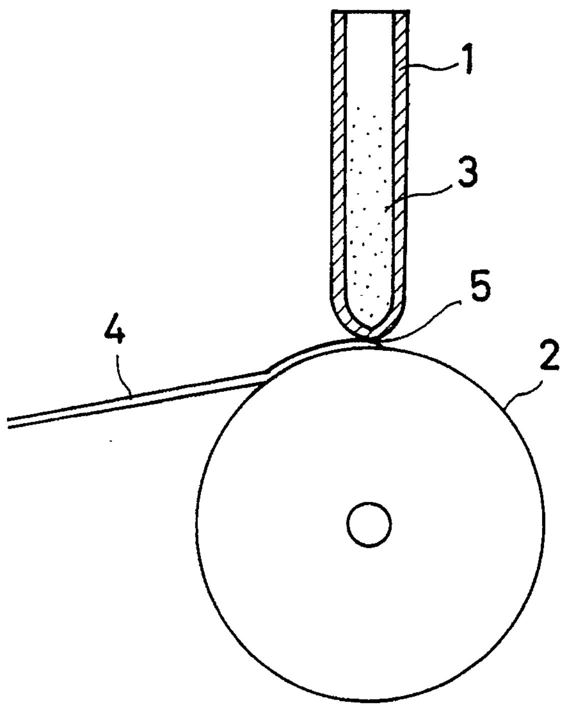
1
3
5
4
2

FIG. 20

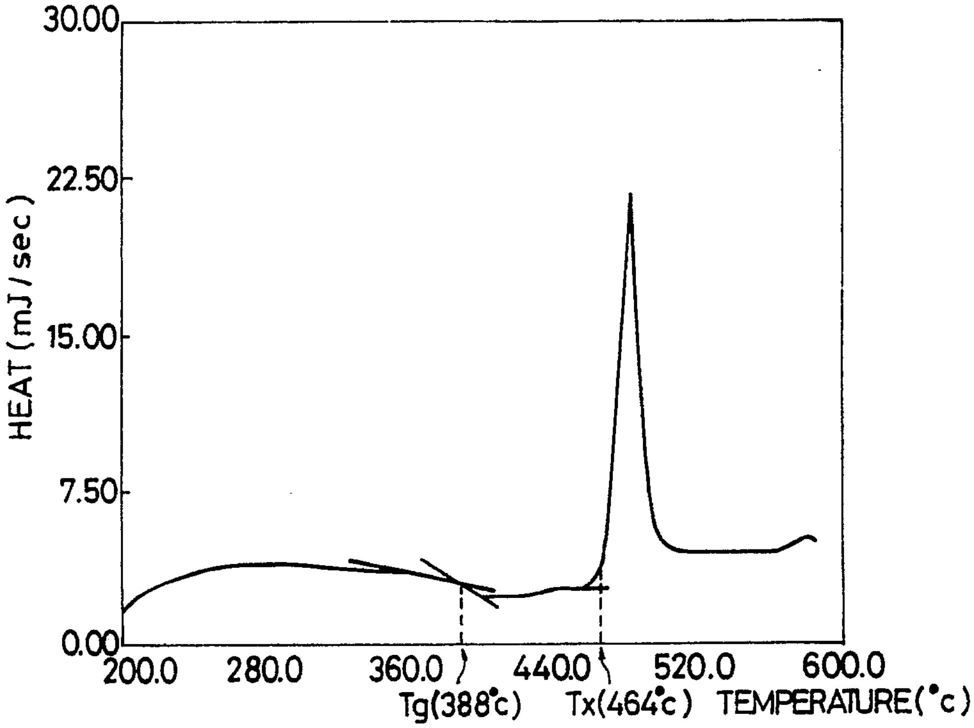
30.00
22.50
15.00
7.50
0.00
HEAT (mJ / sec)
200.0
280.0
360.0
440.0
520.0
600.0
Tg(388°c)
Tx(464°c)
TEMPERATURE(°c)

F I G. 21

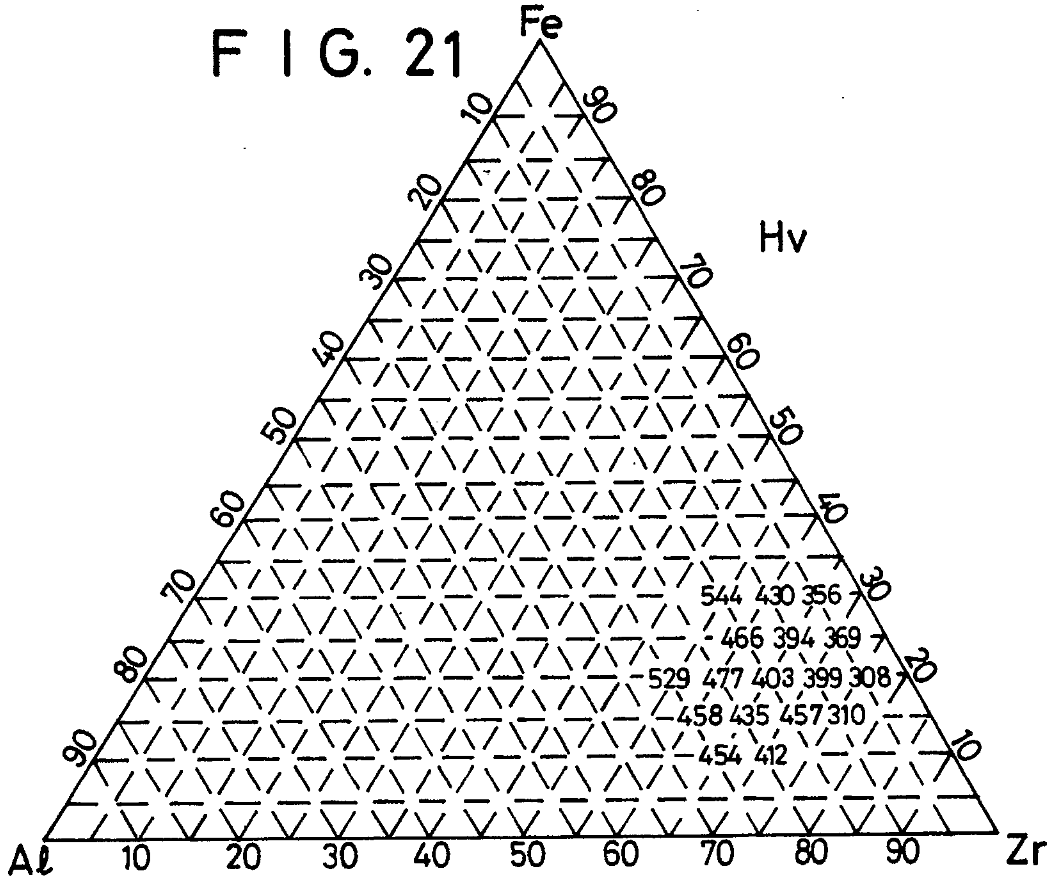
Fe
Hv
544 430 356
466 394 369
529 477 403 399 308
458 435 457 310
454 412
Al
Zr

F I G. 22

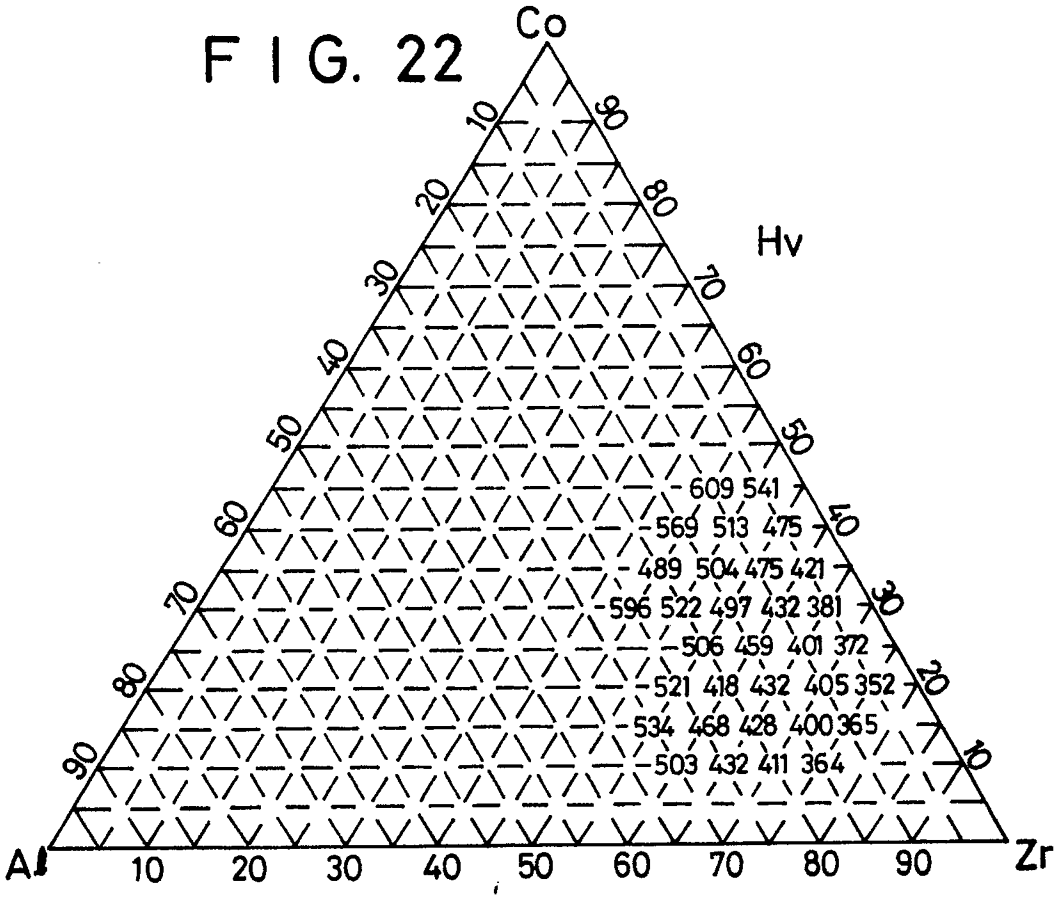
Co
Hv
609 541
569 513 475
489 504 475 421
596 522 497 432 381
506 459 401 372
521 418 432 405 352
534 468 428 400 365
503 432 411 364
Al
Zr